# EUROPEAN PATENT APPLICATION

(11) **EP 4 056 709 A1**
(43) Date of publication of application: **14.09.2022**
(21) Application number: 20884037.1
(22) Date of filing: 28.03.2020
(51) Int. Cl.: C12Q 1/02, C12Q 1/68

(54) **METHOD FOR TESTING DRUG SENSITIVITY OF PATHOGENIC MICROORGANISM**

(30) Priority: 07.11.2019 CN 201911105031; 26.12.2019 CN 201911369806; 18.03.2020 CN 202010192932
(71) Applicant: Hangzhou Dian Medical Laboratory Co., Ltd, Hangzhou, Zhejiang 310030 (CN)
(72) Inventor: REN, Xuyi, Hangzhou, Zhejiang 310030 (CN); CHEN, Shuyun, Hangzhou, Zhejiang 310030 (CN); LV, Jiangfeng, Hangzhou, Zhejiang 310030 (CN); YU, Yuefeng, Hangzhou, Zhejiang 310030 (CN); ZHOU, Jing, Hangzhou, Zhejiang 310030 (CN); YANG, Di, Hangzhou, Zhejiang 310030 (CN); PAN, Caixia, Hangzhou, Zhejiang 310030 (CN); SHI, Hong, Hangzhou, Zhejiang 310030 (CN); YANG, Yichao, Hangzhou, Zhejiang 310030 (CN); CHEN, Yiwang, Hangzhou, Zhejiang 310030 (CN); YUAN, Kai, Hangzhou, Zhejiang 310030 (CN)
(74) Representative: KIPA AB
(86) International application number: PCT/CN2020/081943
(87) International publication number: WO 2021/088306

(57) **Abstract**

The present invention provides methods of identifying pathogens and determining their antimicrobial susceptibility. The methods comprise detecting biomarkers in a test sample, locating the sample in a phylogenetic tree based on biomarker information, obtaining drug susceptibility prediction rules based on the phylogenetic tree positioning of the sample, and determining the drug susceptibility of a pathogen according to the prediction rules. Further provided is an application of the phylogenetic tree in the preparation of pathogen identification and/or drug susceptibility diagnostic product, and a pathogen identification and drug susceptibility diagnostic kit.

## Description

### TECHNICAL FIELD

The present invention pertains to the filed of microbial diagnostic technology, and relates to methods of identifying pathogens and determining their antimicrobial susceptibility, particularly to a method of determining the antimicrobial susceptibility of infectious disease pathogens and applications thereof.

### BACKGROUND

Since the first commercial application of the sulphur antibacterials for the treatment of streptococcal infections in the 1930s, various antibiotics have been widely utilized for infectious disease treatments. It is because of the discovery and application of antibiotics that the human race is no longer helpless in the face of infectious diseases. But over the past years, the misuse or overuse of antibiotics diminishes their curative powers. Once a new antibiotic is introduced, bacteria, by virtue of their natural adaptability, develop resistance to it within two to three years. A 2014 review on antimicrobial resistance (AMR) estimated that by 2050, 10 million people could die each year as a result of AMR, which would become a catastrophic threat to global health and wealth.

Accurate identification of pathogenic bacteria species and accurate testing of drug susceptibility have great reference value for guiding clinical timely and rational drug use. At the same time, it is also crucial to avoid the abuse of antibiotics and effectively control the spread of microbial resistance. At present, detection methods based on culture identification are still the gold standard for the diagnosis of pathogenic bacteria in clinical microbiology laboratories. Relevant technologies include disc diffusion method, E-test test method, broth dilution method, automatic detection equipment, etc. Based on the agar dilution method/broth dilution method (constant method) based on the guidance issued by the American Society for Clinical and Laboratory Standards (NCCLS), the minimum inhibitory effect of the antibacterial agent on the visible growth of the test bacteria was determined after 24-48 hours of in vitro culture. The method of concentration or minimum bactericidal concentration is currently the gold standard for bacterial and fungal susceptibility testing. The advantage of the culture method is that quantitative and qualitative results can be obtained at the same time, and the results are relatively accurate. The disadvantage is that different seeding densities will affect the MIC value when the drug concentration is constant, resulting in tailing and edge effects. Automatic identification and drug susceptibility testing systems for various microorganisms based on the utilization of reaction substrates by substances in microorganisms and the determination of turbidity after culture, such as Vitek 2 compact and Vitek ATB from bioMérieux in France, PhoneixTM-100 from BD in the United States, and Sensititre^{™} ARIS in the United Kingdom, et al. Its essence is a miniaturized broth dilution experiment, which has the advantages of simple operation, objective interpretation, and liberation of manpower, but it is not conducive to promotion due to the high input cost of equipment and related consumables.

The premise of all the above identification and drug susceptibility testing protocols is to obtain single clones after culture. The traditional viable bacteria culture to identify pathogenic bacteria takes 2-3 days, and the drug sensitivity incubation time is 24-48 hours. The total time for identification and drug susceptibility is usually 3-5 days. During this period, the patient's condition may have changed, and the test report has lost value for the patient's treatment, thus greatly reducing the clinical enthusiasm for the application of microbial testing technology, and the subsequent choice of empirical medication is also the most direct cause of antibiotic abuse.

Matrix-assisted laser desorption/ionization time-of-flight mass spectrometry (MALDI-TOF MS) is a new type of soft ionization biological mass spectrometry developed in recent years. It identifies macromolecules such as proteins and polypeptides through the time-of-flight required for the molecules formed by the co-crystallization of the sample and the matrix after laser irradiation to pass through the vacuum tube, and has been widely used in the field of microbial identification. However, a mature detection methodology has not been established in the field of drug susceptibility diagnosis. At the same time, because the detection targets of this method are macromolecules such as proteins and peptides, macromolecules are often not the most direct participants in cell metabolism, which limits the improvement of its detection sensitivity and specificity.

Molecular diagnostic technology is a revolutionary technology in the field of pathogen identification in that it is rapid, highly sensitive and simultaneous detection of multiple pathogens. Various rapid pathogen identification products based on Sanger sequencing, Pyrosequencing, quantitative PCR analysis, Microarray Analysis, etc. have been developed and commercialized. The increasingly inexpensive whole genome sequencing (WGS) technology has becoming a universal and unbiased pathogen detection method for infectious disease diagnostic, with applications including pathogen identification, outbreak investigation and health care surveillance. However, use of WGS technology and AMR genes analysis for antimicrobial resistance prediction has always had challenges and technical bottleneck. The main concern is the lack of understanding of the genotype-to-phenotype correspondence. Firstly, the emergence of resistance genes is not always consistent with the resistance phenotype, but is also regulated by the genetic environment in which the genes are located. Secondly, the detection of drug resistance genes by PCR technology is prone to false positive results. Thirdly, the detection and analysis of known resistance genes must be accompanied by the limitation of not being able to detect new or atypical resistance mechanisms. In summary, at present there is still no ideal microbial diagnostic product of infectious diseases which offers rapid, robust, cost-effective, highly sensitive, simultaneous detection of identification and drug susceptibility directly from clinical samples, and provides informative and accurate antimicrobial susceptibility diagnostic results within hours.

### SUMMARY

To overcome aforesaid disadvantages of the prior art, the present invention has designed a kind of method, and applications thereof, simultaneously identifying pathogens and determining their antimicrobial susceptibility directly from clinical samples based on detection of biomarkers. Further, the present invention provides a pathogen identification and drug susceptibility diagnostic kit based on LC-MS and/or WGS technology; non-diagnostic methods using said kit, e.g., pharmaceutical research.

For reaching above-mentioned objects of the invention, LC-MS and WGS technologies are employed. The LC-MS technology effectively combines the advantages of liquid chromatography for compounds separation and mass spectrometry for high resolution, superior qualitative and quantitative compound analysis. The metabolic fingerprints are accuratedly separated, identified and quantified to distinguish different pathogens, and located to different metabolic pathways to determine the antimicrobial susceptibility. The WGS technology, particularly the third-generation sequencing technology, has outstanding advantages of real-time detection without amplification, Mb-level sequencing read length, precise genome assembly, affordable and portable instrument, *etc.* and allows for unbiased and comprehensive analysis the genome and antibiotic resistance determinants.

In above-mentioned method, both LC-MS and WGS technologies can independently and simultaneously realize the pathogen identification and drug susceptibility determination. The above-mentioned method based on LC-MS technology, using culture colonies as the test sample, takes advantage of rapid sample preparation (15 mins per batch) and detection (5 mins per sample), and thus offers a microbial susceptibility test report within 30 minutes. The above-mentioned method based on WGS technology, using clinical specimen as the test sample, breaks through the bottleneck of low culture positive rate and long culture time, and achieves high-throughput, highly sensitive pathogen detection directly from clinical samples within 6 hours. The present invention provides the pathogen identification and drug susceptibility determination methods based on both LC-MS and WGS technologies, and allows the users to choose an optimal method according to different sample types and timeliness requirements.

In one aspect, the present invention features methods of pathogen identification and/or drug susceptibility determination. The methods comprise detecting biomarkers in a test sample, locating the sample in a phylogenetic tree based on biomarker information, obtaining drug susceptibility prediction rules based on the phylogenetic tree positioning of the sample, and determining the drug susceptibility of a pathogen according to the prediction rules.

Further, the biomarker information is metabolic fingerprints and/or resistance determinant nucleic acid sequences of a pathogen.

Further, the metabolic fingerprints are feature information of metabolites detected by mass spectrometry, preferably, the feature information is one or more of mass-to-charge ratio, retention time, and species abundance of the metabolites.

The method of pathogen identification and/or drug susceptibility determination described in the present invention can be diagnostic or non-diagnostic; said method applies a combination of phylogenetic tree, biomarker information, and prediction rules to determine the species and drug susceptibility of a pathogen; wherein the positioning of the sample on the phylogenetic tree is used for species identification; wherein the the positioning of the sample on the phylogenetic tree, combined with biomarker information and prediction rules, are used for susceptibility determination.

Further, the phylogenetic tree is obtained by liquid chromatography-tandem mass spectrometry technology and/or whole genome sequencing technology.

Further, the type of phylogenetic tree includes, but is not limited to: a metabolic spectrum phylogenetic tree constructed based on the species and amounts of metabolites, a whole genome phylogenetic tree constructed based on SNPs and InDels, and/or a core genome phylogenetic tree constructed based on antimicrobial resistance determinants and their upstream regulatory sequences.

Further, the biomarker information includes, but is not limited to: the retention time and mass-to-charge ratio of amino acids, organic acids, fatty acids, sugar derivatives and other metabolites, the nucleic acid sequences of antibiotic resistance genes, plasmids, chromosomal housekeeping genes, insertion sequences, transposons, integrons and other antimicrobial resistance determinants.

Further, the drug susceptibility prediction rules include, but are not limited to: different metabolite-based prediction rules are applied for different branches of the metabolic spectrum phylogenetic tree, and/or different sequence-based prediction rules are applied for different branches of the genomic phylogenetic tree.

Further, the metabolites are water-soluble molecules with a mass-to-charge ratio between 50-1500 Da and a minimum abundance value of 2000.

Further, the deviation range of the retention time is ±0.5min, and the deviation range of the mass-to-charge ratio is ±0.05Da.

Further, the phylogenetic tree is a rootless tree constructed according to the k-mer frequency in whole genome sequence of representative clones.

Further, the antimicrobial resistance determinants include, but are not limited to:
*abarmA, abAPH(3')-Ia, abOX4239, abNDM-10, abgyrA, abSUL-1, abSUL-2, abSUL-3, kpCTX-M-65, kpTEM-1b, kpIMP-4, kpKPC-2, kprmtB, kpAAC(3')-Iid, kpQNR-S, kpgyrA, kpparC, kptetA, kptetD, kpSUL-1, kpSUL-2, kpSUL-3, ecrmtB, ecAAC(3')-Iid, ecgyrA, ectetA, ectetB, ecSUL-1, ecSUL-2, ecSUL-3, ecIMP-4, ecNDM-5, ecTEM-1b, ecCTX-M-14, ecCTX-M-55, ecCTX-M-65, ecCMY, paTEM-1b, paGES-1, paPER-1, paK-PC-2, paOXA-246, parmtB, paAAC(3)-Iid, paAAC(6')-IIa, paVIM-2, pagyrA, efermB, eftetM, eftetL, efparC, efANT(6')-Ia, stmecA, stmsrA, stermA, stermB, stermC, strpoB, stgyrA, stAAC(6')-APH(2"), stdfrG, sttetK, sttetL, stcfrA, spbpb1la, sppbp2x, spbpb2b, spdfr, sptetM, spermB, spgyrA, aat1a, acc1, adp1, mpib, sya1, vps13, zwf1b, fcy2, fur1, fca1, erg11, erg3, tac1, cdr1, cdr2, mdr1, pdr1, upc2a, fks1hs1, jks1hs2, fks2hs1, jks2hs2,* and the combinations thereof.

Further, the metabolite-based prediction rules include, but are not limited to:
1) Use independent prediction rules in the drug susceptibility determination of non-fermentative Gram-negative bacteria: when the test sample contains metabolic fingerprints of a specific clone, follow the principle that the drug resistance profile predicted by metabolic fingerprints is preferred over phylogenetic tree interpretation; when the test sample is located in the susceptibility branch of the metabolic spectrum phylogenetic tree, follow the principle that the drug resistance profile inferred by the phylogenetic tree is preferred over metabolic fingerprints and the sample is directly determined to be susceptible to all β-lactam antibiotics;
2) Use independent prediction rules in the drug susceptibility determination of Enterobacteriaceae: when the test sample contains metabolic fingerprints of a specific clone, follow the principle that the drug resistance profile predicted by metabolic fingerprints is preferred over phylogenetic tree interpretation; when the test sample is located in the susceptibility branch of the metabolic spectrum phylogenetic tree, follow the principle that the drug resistance profile inferred by the phylogenetic tree is preferred over metabolic fingerprints and the sample is directly determined to be susceptible to β-lactams, β-lactamase inhibitors and cephamycins;
3) Use independent prediction rules in the drug susceptibility determination of Gram-positive cocci: when the test sample contains metabolic fingerprints of a specific clone, follow the principle that the drug resistance profile predicted by metabolic fingerprints is preferred over phylogenetic tree interpretation; when the test sample is located in the susceptibility branch of the metabolic spectrum phylogenetic tree, follow the principle that the drug resistance profile inferred by the phylogenetic tree is preferred over metabolic fingerprints and the sample is directly determined to be susceptible to penicillins, macrolides, lincosamides, quinolones, aminoglycosides, glycopeptides and oxazolidinones; when the test sample is identified as *Enterococcus faecalis* and has the metabolic fingerprints of a sequence type 4 *Enterococcus faecalis* clone, the sample is directly determined to be resistant to penicillins;

Preferably, for *Staphylococcus spp.,* when the test sample is located in the mecA-positive branch of the metabolic spectrum phylogenetic tree, follow the principle that the drug resistance profile inferred by the phylogenetic tree is preferred over metabolic fingerprints and the sample is directly determined to be resistant to penicillins, cefoxitin and quinolones; when the test sample is located in the mecA-negative branch of the metabolic spectrum phylogenetic tree, follow the principle that the drug resistance profile inferred by the phylogenetic tree is preferred over metabolic fingerprints and the sample is directly determined to be susceptible to penicillins and cefoxitin; when the test sample is located in the susceptibility branch of the metabolic spectrum phylogenetic tree, follow the principle that the drug resistance profile inferred by the phylogenetic tree is preferred over metabolic fingerprints and the sample is directly determined to be susceptible to penicillins, cefoxitin, macrolides, lincosamides, quinolones, aminoglycosides, glycopeptides and oxazolidinones; When the test sample is located in a branch other than the above-specified branches, follow the principle that the drug resistance profile is determined solely by metabolic fingerprints.

Preferably, for *Enterococcus faecalis,* when the test sample has metabolic fingerprints of a specific clone, follow the principle that the drug resistance profile inferred by metabolic fingerprints is preferred over the phylogenetic tree interpretation; specifically, when the test sample has the metabolic fingerprints of a sequence type 4 Enterococcus faecalis clone, the sample is directly determined to be resistant to penicillins;
4) Use independent prediction rules in the drug susceptibility determination of *Streptococcus pneumoniae:* when the test sample contains metabolic fingerprints of a specific clone, follow the principle that the drug resistance profile predicted by metabolic fingerprints is preferred over phylogenetic tree interpretation; when the test sample is identified as *Streptococcus pneumoniae* and has the metabolic fingerprints of a *Streptococcus pneumoniae* clone with altered penicillin-binding protein patterns, the sample is directly determined to be resistant to penicillins;
and/or, 5) Use independent prediction rules in the drug susceptibility determination of Fungi: strictly follow the principle that the drug resistance profile of a fungal strain is inferred on the basis of its closest relatives in the metabolic spectrum phylogenetic tree.

Further, the sequence-based prediction rules include, but are not limited to:
1) Resistance of Enterobacteriaceae to carbapenems and quinolones is determined by the positioning of a test sample in the genomic phylogenetic tree, following the principle that the drug resistance profile predicted by phylogenetic tree interpretation is preferred over antimicrobial resistance determinants analysis; resistance of Enterobacteriaceae to aminoglycosides, tetracyclines, sulfonamides, β-lactams except carbapenems is determined by antimicrobial resistance determinants analysis;
2) Resistance of non-fermentative Gram-negative bacteria to cephalosporins and carbapenems is determined by the positioning of a test sample in the genomic phylogenetic tree, following the principle that the drug resistance profile predicted by phylogenetic tree interpretation is preferred over antimicrobial resistance determinants analysis; resistance of non-fermentative Gram-negative bacteria to aminoglycosides, tetracyclines, sulfonamides, quinolones and β-lactamase inhibitors is determined by antimicrobial resistance determinants analysis;
3) Resistance of Gram-positive cocci to penicillin, ampicillin, oxacillin and cefoxitin is determined by the positioning of a test sample in the genomic phylogenetic tree, following the principle that the drug resistance profile predicted by phylogenetic tree interpretation is preferred over antimicrobial resistance determinants analysis; resistance of Gram-positive cocci to macrolides, lincosamides, aminoglycosides, quinolones, glycopeptides and oxazolidinones is determined by antimicrobial resistance determinants analysis;
4) Resistance of *Streptococcus pneumoniae* to penicillins and cephalosporins is determined by the positioning of a test sample in the genomic phylogenetic tree, following the principle that the drug resistance profile predicted by phylogenetic tree interpretation is preferred over antimicrobial resistance determinants analysis; resistance of *Streptococcus pneumoniae* to macrolides, lincosamides, aminoglycosides, quinolones, glycopeptides and oxazolidinones is determined by antimicrobial resistance determinants analysis;
and/or, 5) Resistance of Fungi to triazoles and amphotericin B formulations is determined by the positioning of a test sample in the genomic phylogenetic tree, following the principle that the drug resistance profile predicted by phylogenetic tree interpretation is preferred over antimicrobial resistance determinants analysis; resistance of Fungi to echinocandins is determined by antimicrobial resistance determinants analysis.

In the present invention, bacterial pathogens are divided into 4 categories: enterobacteriaceae, non-fermentative Gram-negative bacteria, Gram-positive cocci, and fastidious bacteria according to their Gram-staining properties, morphological characteristics, carbohydrate fermentation patterns, and nutritional requirements for growth, which is in agreement with their phylogenetic distance and differentiated clinical medication and accounts for more than 90% of clinical pathogens. Those skilled in the art can understand that such representative species as the enterobacteriaceae *Klebsiella pneumonia,* the non-fermenting *Acinetobacter baumannii,* the Gram-positive cocci *Enterococcus faecalis* and *Staphylococcus aureus,* the fastidious *Streptococcus pneumoniae,* and the fungus *Candida tropicalis* are suitable for demonstrating the applicability of the pathogen identification and drug susceptibility diagnostic method. Other bacterial and fungal species can refer to the method and prediction rules of the present invention.

In another aspect, the present invention also provides an application of the phylogenetic tree in the preparation of pathogen identification and/or drug susceptibility diagnostic product, wherein the phylogenetic tree is obtained by liquid chromatography tandem mass spectrometry technology and/or whole genome sequencing technology.

Further, the phylogenetic tree is selected from the group consisting of a metabolic spectrum phylogenetic tree constructed based on the species and amounts of metabolites, a whole genome phylogenetic tree constructed based on SNPs and InDels, and a core genome phylogenetic tree constructed based on antimicrobial resistance determinants and their upstream regulatory sequences.

Further, the pathogen identification and/or drug susceptibility diagnostic product comprises reagents and equipment for obtaining the biomarker information in a test sample.

Further, the equipment for obtaining the biomarker information is liquid chromatography-tandem mass spectrometry and/or whole genome sequencing devices.

Further, the reagents for obtaining the biomarker information are the pathogen identification and drug susceptibility diagnostic kit based on liquid chromatography-tandem mass spectrometry, and/or the pathogen identification and drug susceptibility diagnostic kit based on whole genome sequencing technology.

In another aspect, the present invention provides a pathogen identification and drug susceptibility diagnostic kit, comprising:
KIT1: pathogen identification and drug susceptibility diagnostic kit based on liquid chromatography-tandem mass spectrometry; and/or,
KIT2: pathogen identification and drug susceptibility diagnostic kit based on whole genome sequencing technology.

Further, the pathogen identification and drug susceptibility diagnostic kit comprises the phylogenetic trees of pathogens.

Further, the pathogen identification and drug susceptibility diagnostic kit based on liquid chromatography-tandem mass spectrometry comprise bacterial standards, fungal standards, extraction buffer and resuspension buffer.

Further, the pathogen identification and drug susceptibility diagnostic kit based on whole genome sequencing technology comprise cell lysis reagents, primer mixture, target enrichment reagents, library preparation reagents, native barcoding reagents and sequencing reagents.

Further, the bacterial standards are a methanol solution containing 128 ng/mL 5-fluorocytosine, pre-cooled at 2-8°C.

Further, the fungal standards are a methanol-water (v/v 4:1) solution containing 126 ng/mL ampicillin, pre-cooled at 2-8 °C.

Further, the extraction buffer is a methanol-acetonitrile mixture (v/v 2:1), pre-cooled at -20 to -80°C.

Further, the resuspension buffer is a water-acetonitrile-formic acid mixture (v/v/v 98:2:0.05), pre-cooled at 2~8°C.

Further, the cell lysis reagents contain 0.02% (m/v) saponin.

Further, the applicable sample type of the pathogen identification and drug susceptibility diagnostic kit based on liquid chromatography-tandem mass spectrometry is culture colony; the applicable sample types of the pathogen identification and drug susceptibility diagnostic kit based on whole genome sequencing technology are clinical specimen or colony cultures.

In yet another aspect, the present invention provides the application of the above-mentioned pathogen identification and drug susceptibility diagnostic kit in pathogen species identification and antimicrobial susceptibility determination.

### DESCRIPTION OF DRAWINGS

FIGS. 1a to 1c are a metabolic spectrum phylogenetic tree constructed by 82 metabolic fingerprints of 24 bacterial species for bacterial identification.
FIGS. 2a to 2b show the distribution of 16 blind samples in the metabolic spectrum phylogenetic tree for bacterial identification. The branches of the phylogenetic tree representing different species are indicated by different colors on the left, where black represents blind samples.
FIGS. 3a to 3b are a metabolic spectrum phylogenetic tree constructed by 87 representative *Acinetobacter baumannii* metabolic fingerprints for susceptibility inference.
FIGS. 4a to 4b show the distribution of 16 blind *Acinetobacter baumannii* samples in the metabolic spectrum phylogenetic tree for susceptibility inference. The branches of the phylogenetic tree representing different resistance profiles are indicated by different colors on the left, where black represents blind samples.
FIG. 5 is a metabolic spectrum phylogenetic tree constructed by 36 representative *Enterococcus faecalis* metabolic fingerprints for susceptibility inference.
FIG. 6 shows the distribution of 6 blind *Enterococcus faecalis* samples in the metabolic spectrum phylogenetic tree for susceptibility inference. The branches of the phylogenetic tree representing different resistance profiles are indicated by different colors on the left, where black represents blind samples.
FIG. 7 is a metabolic spectrum phylogenetic tree constructed by 18 representative *Streptococcus pneumoniae* metabolic fingerprints for susceptibility inference.
FIG. 8 shows the distribution of 2 blind *Streptococcus pneumoniae* samples in the metabolic spectrum phylogenetic tree for susceptibility inference. The branches of the phylogenetic tree representing different resistance profiles are indicated by different colors on the left, where black represents blind samples.
FIGS. 9a to 9b are a metabolic spectrum phylogenetic tree constructed by 115 metabolic fingerprints representative of 22 fungal species for fungal identification.
FIGS. 10a to 10b show the distribution of 8 blind samples in the metabolic spectrum phylogenetic tree for fungal identification. The branches of the phylogenetic tree representing different species are indicated by different colors on the left, where black represents blind samples.
FIG. 11 shows the distribution of 6 blind *Candida tropicalis* samples in the metabolic spectrum phylogenetic tree for susceptibility inference. The branches of the phylogenetic tree representing different resistance profiles are indicated by different colors on the left, where blue indicates azole-susceptible, yellow indicates azole-resistant, and black represents blind samples.
FIGS. 12a to 12c are a genomic phylogenetic tree constructed by 165 representative *Klebsiella pneumoniae* genomes for susceptibility inference. The branches of the phylogenetic tree representing different resistance profiles are indicated by different colors on the right, where yellow indicates ST11 clone, red indicates ST15 clone, and blue indicates susceptible to all antibiotics (including ST23 clone).
FIG. 13 is a genomic phylogenetic tree constructed by 93 representative *Staphylococcus aureus* genomes for susceptibility inference. The branches of the phylogenetic tree representing different resistance profiles are indicated by different colors on the right.
FIG. 14 shows the genomic phylogenetic tree constructed by 25 representative *Streptococcus pneumoniae* genomes and the distribution of 2 blind samples in the genomic phylogenetic tree for for susceptibility inference. The branches of the phylogenetic tree representing different resistance profiles are indicated by different colors on the right, where yellow indicates susceptible to all antibiotics, blue indicates penicillin-resistant, and black represents blind samples.
FIG. 15 is a genomic phylogenetic tree constructed by 107 representative *Candida albicans* genomes for susceptibility inference. The branches of the phylogenetic tree representing different resistance profiles are indicated by different colors on the right, where red indicates azole-resistant, yellow indicates 5-fluorocytosine-resistant, green indicates echinocandins-resistant, gray indicates amphotericin-B-intermediate, and blue indicates susceptible to all antibiotics.
FIG. 16 shows the distribution of a respiratory sputum sample in the genomic phylogenetic tree of *Acinetobacter baumannii.*
FIG. 17 shows the distribution of a respiratory sputum sample in the genomic phylogenetic tree of *Klebsiella pneumoniae.*

### DETAILED DESCRIPTION

The following embodiments further illustrate content of the present invention, but should not be construed as a limitation on the present invention. Modifications and substitutions made to the methods, steps, or conditions of the present invention without departing from the spirit and essence of the present invention shall fall within the scope of the present invention. Unless otherwise specified, in each table (tables 1 to 24), 'R' indicates drug-resistant, 'S' indicates drug-susceptible, blank means no interpretation.

### Example 1: Construction and validation of a metabolic spectrum phylogenetic tree database for bacterial identification based on LC-MS

### 1. Method

Step 1. Sample collection and identification: 430 clinical isolates were collected from 42 hospitals across china in a period between May 2017 and July 2019. All isolates were subjected to Sanger sequencing or the third-generation whole genome sequencing, as the gold standard for species identification.

Step 2. Preparation of bacterial suspension: Each test isolate was inoculated on Columbia sheep blood agar plate, and after culturing overnight, an appropriate amount of colonies was scraped into a sterile saline tube with a disposable inoculation loop to prepare a homogeneous bacterial suspension.

Step 3. Cell breakage: An equal volume of bacterial standards was added to 180 µL of bacterial suspension, and sonicated at 80 Hz for 5 min.

Step 4. Extraction and concentration of metabolites: 340 µL of the sonication product obtained in step 3 was transferred to a 1.5 ml centrifuge tube, mixed with an equal volume of extraction buffer, shaked on ice for 3 min, and centrifuged quickly for 3-5 seconds. The liquid in the tube was spin down to the bottom of the tube and dried using a nitrogen blower.

Step 5. Mass spectrometry detection: The residue obtained in step 4 was resuspended with 140 µL of resuspension buffer, vortexed, centrifuged at high speed for 5 min. The supernatant was transferred to a new centrifuge tube, and centrifuged at high speed for 5 min. 100 µL of the supernatant was transferred to the sample introduction system of LC-MS, and 4 µL of the sample was injected each run for detection and analysis; preferably, the high-resolution LC-MS is Waters Q-TOF Synapt G2-Si quadrupole-time-of-flight mass spectrometer. The retention was achieved in gradient elution reversed-phase chromatography under the conditions as follows: water-acetonitrile-formic acid was used as the mobile phase system, the flow rate of the mobile phase was 0.4 ml/min, and the column temperature was 40 °C; the chromatographic column was a Waters HSS T3 column with a particle size of 1.8 µm, an inner diameter of 2.1 mm, and a column length of 100 mm; Mass spectrometry detection adopts electrospray ionization source (ESI), positive ion mode, multiple reaction monitoring scan mode (MRM) and MSeContinnum data independent acquisition mode.

### 2. Bioinformatics analysis and database construction

(1) Biomarker screening: Progenesis QI software was used to generate peak alignment, peak extraction, compound identification, and normalization on the raw data collected by mass spectrometry, and output feature information such as mass-to-charge ratio (m/z), retention time, and abundance. Bacteria of different species were categorized into different groups for principal component analysis, with parameters for screening biomarkers set as: fold change>10, VIP>1, p value<0.05, CV<30%. The following 258 biomarkers were screened out: 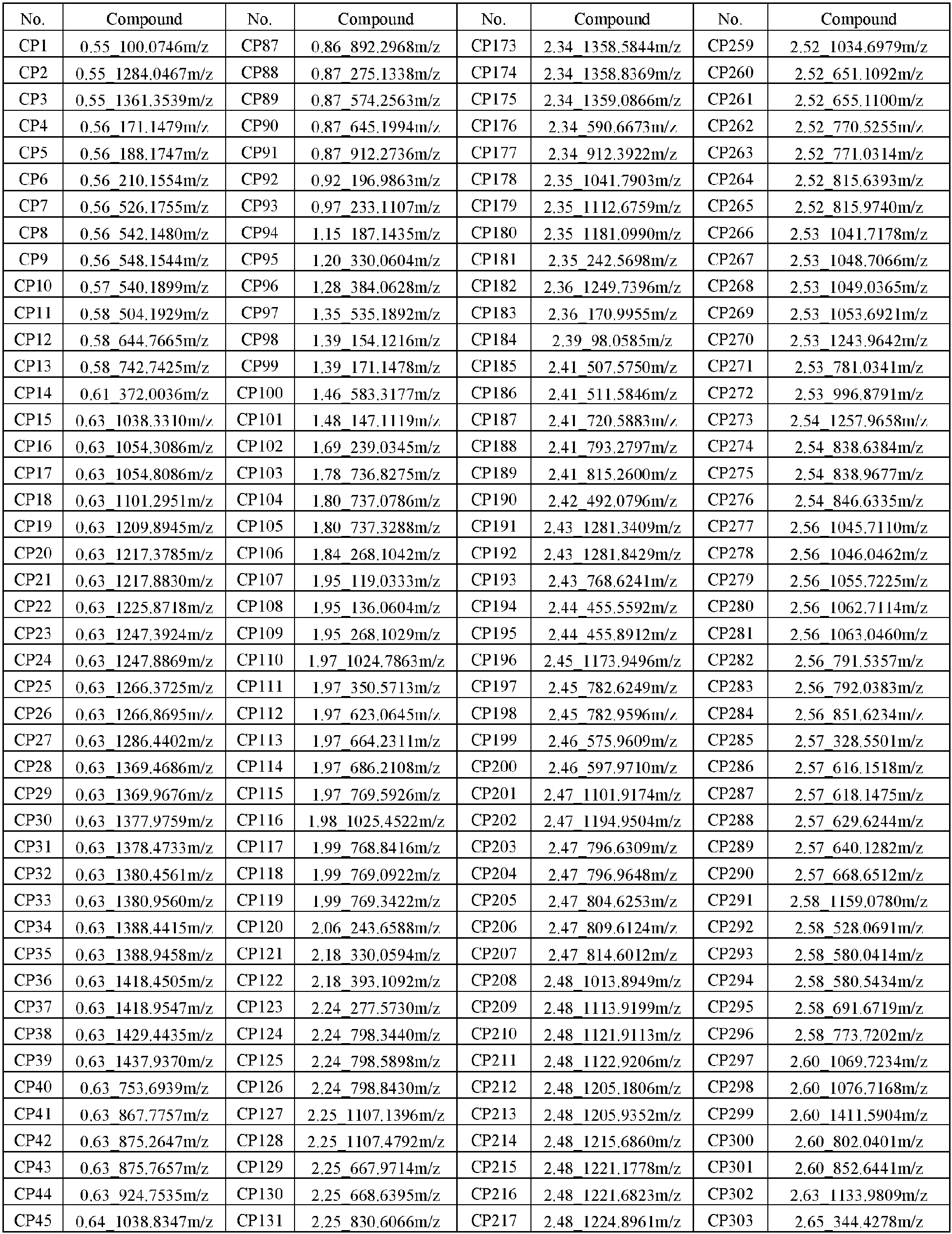 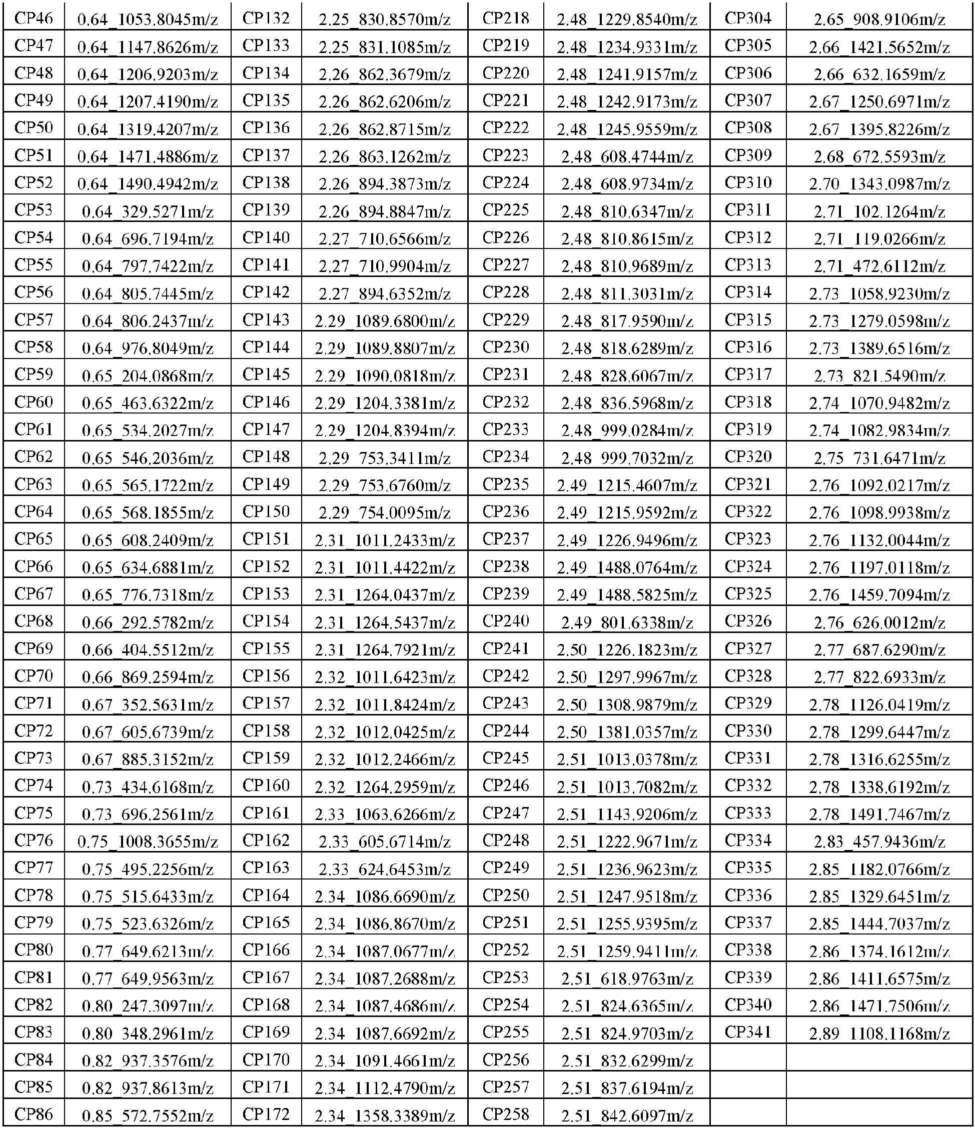
(2) Construction of a metabolic spectrum phylogenetic tree database: To optimize the representativeness and reproducibility of the database, a variety of enterobacteriaceae bacteria, non-fermenting bacteria and Gram-positive cocci were evenly covered and at least 10 isolates from each hospital were emplyed for metabolic fingerprints profiling test. To optimize the resolution of the database, closely-related species such as *Acinetobacter baumannii, Acinetobacter nosocomialis* and *Acinetobacter pittii, Klebsiella pneumoniae, Klebsiella variicola* and *Klebsiella quasipneumoniae* which could not be accurately distinguished by the automated identification system such as VITEK2, were selected and added to the database.

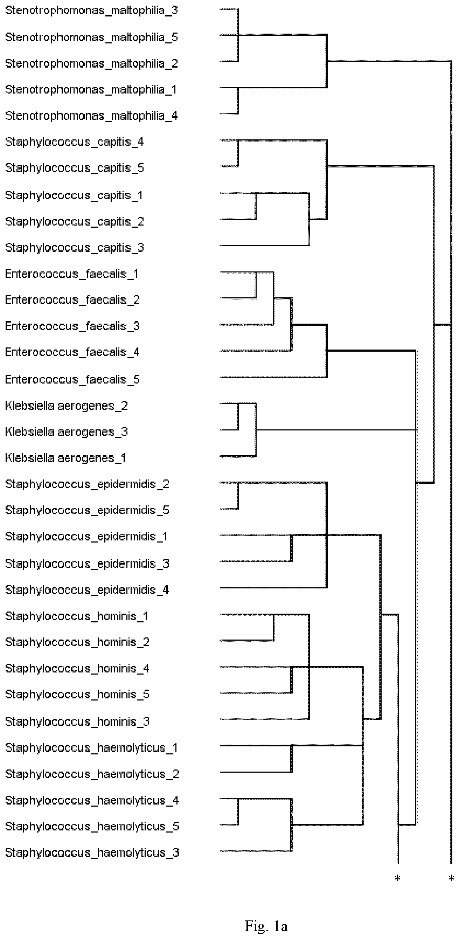

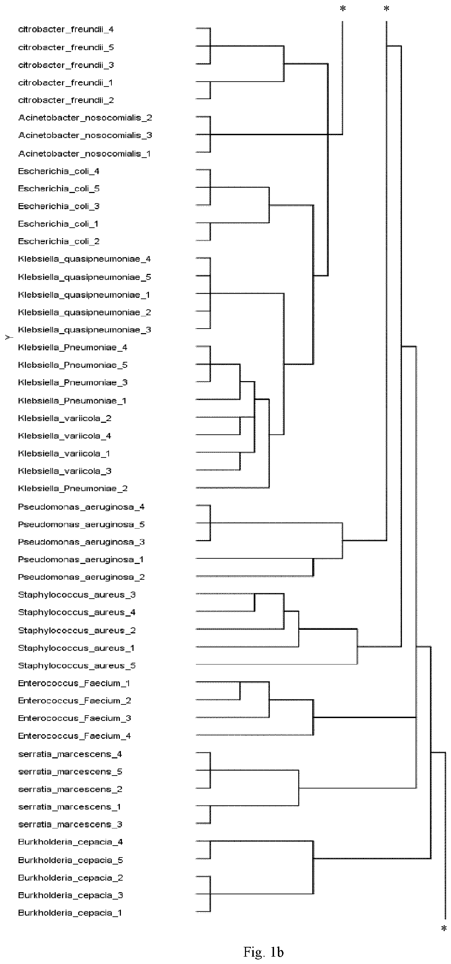

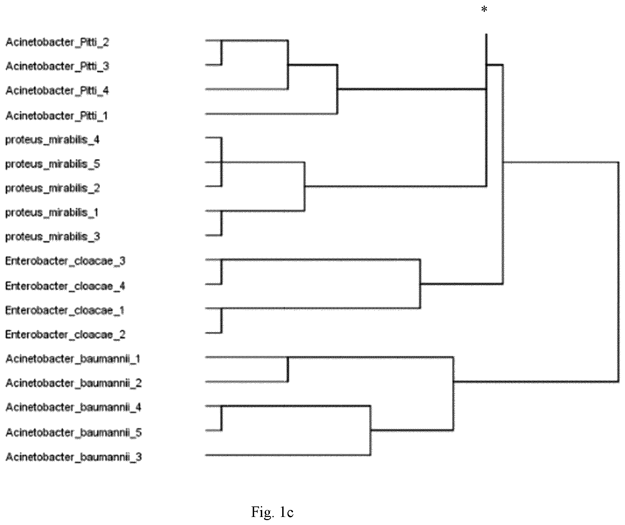

Through biomarker analysis, identical clones were merged and 82 representative clones were selected for the database. The feature information of the biomarkers (retention time, mass-to-charge ratio, abundance) was imported into the software IBM SPSS Statistics 23, the retention time and mass-to-charge ratio information used as variable names, the compound abundance used as variable value. Using the analysis mode of 'systematic cluster', a dendrogram was generated, as shown in Figure 1.

### 3. Database validation and result interpretation

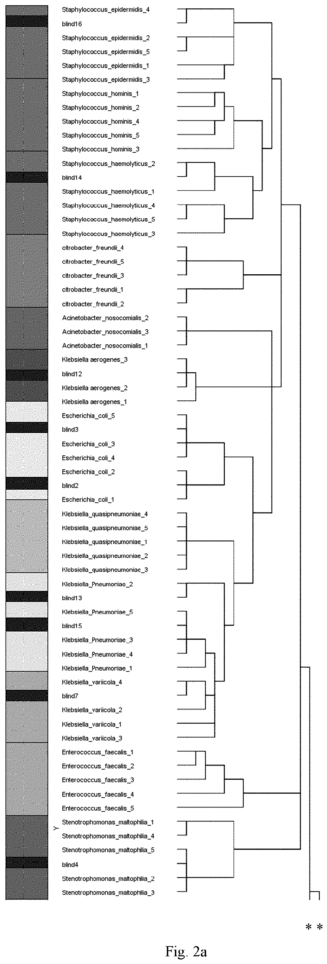

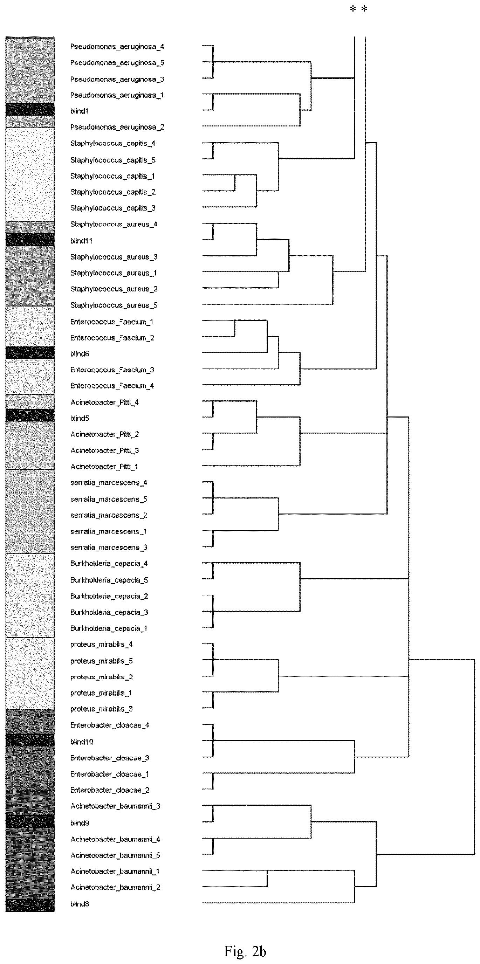

(1) Blind test: The metabolic fingerprints of 16 blinds were imported into the software IBM SPSS Statistics 23 for cluster analysis. The species of each blind was determined based on its positioning in the phylogenetic tree. As is shown in Figure 2, the branches of the phylogenetic tree representing different species are indicated by different colors on the left, where black represents blind samples.
(2) Result interpretation: The species of 16 blinds were predicted based on their positioning in the phylogenetic tree and the corresponding prediction rules. As is shown in Table 1, identification results predicted by method of this invention were in a 100% agreement with the gold standard (sequencing method) result. Notably, closely relates species such as *Acinetobacter baumannii* and *Acinetobacter pittii,* which can not be distinguished by automated identification systems such as VITEK2, were identified in complete agreement with the gold standard method.

**Table 1**

| blind sample | result by method of this invention | result by gold standard (sequencing) | agreement |
|---|---|---|---|
| blind-1 | *Pseudomonas aeruginosa* | *Pseudomonas aeruginosa* | yes |
| blind-2 | *Escherichia coli* | *Escherichia coli* | yes |
| blind-3 | *Escherichia coli* | *Escherichia coli* | yes |
| blind-4 | *Stenotrophomonas maltophilia* | *Stenotrophomonas maltophilia* | yes |
| blind-5 | *Acinetobacter pittii* | *Acinetobacter pittii* | yes |
| blind-6 | *Enterococcus faecium* | *Enterococcus faecium* | yes |
| blind-7 | *Klebsiella variicola* | *Klebsiella variicola* | yes |
| blind-8 | *Acinetobacter baumannii* | *Acinetobacter baumannii* | yes |
| blind-9 | *Acinetobacter baumannii* | *Acinetobacter baumannii* | yes |
| blind-10 | *Enterobacter cloacae* | *Enterobacter cloacae* | yes |
| blind-11 | *Staphylococcus aureus* | *Staphylococcus aureus* | yes |
| blind-12 | *Klebsiella aerogenes* | *Klebsiella aerogenes* | yes |
| blind-13 | *Klebsiella pneumoniae* | *Klebsiella pneumoniae* | yes |
| blind-14 | *Staphylococcus haemolyticus* | *Staphylococcus haemolyticus* | yes |
| blind-15 | *Klebsiella pneumoniae* | *Klebsiella pneumoniae* | yes |
| blind-16 | *Staphylococcus epidermidis* | *Staphylococcus epidermidis* | yes |

### Example 2: Construction and validation of a metabolic spectrum phylogenetic tree database for Acinetobacter baumannii based on LC-MS

*Acinetobacter baumannii* was selected as the representative of Gram-negative bacteria to illustrate the way a metabolic spectrum phylogenetic tree for drug susceptibility determination is constructed. Those skilled in the art can understand that this method is applicable to other Gram-negative bacteria including enterobacteriaceae and non-fermentative bacteria.

### 1. Method

Step 1. Drug susceptibility verification: The *Acinetobacter baumannii* isolates were tested for drug susceptibility using bioMérieux Vitek2 Compact System and AST -N335 cards, and the results were used as the gold standard (culture-based AST).

Step 2. Preparation of bacterial suspension: Each test isolate was inoculated on Columbia sheep blood agar plate, and after culturing overnight, an appropriate amount of colonies was scraped into a sterile saline tube with a disposable inoculation loop to prepare a homogeneous bacterial suspension.

Step 3. Cell breakage: an equal volume of bacterial standards was added to 180 µL of bacterial suspension, and sonicated at 80 Hz for 5 min.

Step 4. Extraction and concentration of metabolites: 340 µL of the sonication product obtained in step 3 was transferred to a 1.5 ml centrifuge tube, mixed with an equal volume of extraction buffer, shaked on ice for 3 min, and centrifuged quickly for 3-5 seconds. The liquid in the tube was spin down to the bottom of the tube and dried using a nitrogen blower.

Step 5. Mass spectrometry detection: the residue obtained in step 4 was resuspended with 140 µL of resuspension buffer, vortexed, centrifuged at high speed for 5 min. The supernatant was transferred to a new centrifuge tube, and centrifuged at high speed for 5 min. 100 µL of the supernatant was transferred to the sample introduction system of LC-MS, and 4 µL of the sample was injected each run for detection and analysis; preferably, the high-resolution LC-MS is Waters Q-TOF Synapt G2-Si quadrupole-time-of-flight mass spectrometer. The retention was achieved in gradient elution reversed-phase chromatography under the conditions as follows: water-acetonitrile-formic acid was used as the mobile phase system, the flow rate of the mobile phase was 0.4 ml/min, and the column temperature was 40 °C; the chromatographic column was a Waters HSS T3 column with a particle size of 1.8 µm, an inner diameter of 2.1 mm, and a column length of 100 mm; Mass spectrometry detection adopts electrospray ionization source (ESI), positive ion mode, multiple reaction monitoring scan mode (MRM) and MSeContinnum data independent acquisition mode.

### 2. Bioinformatics analysis and database construction

(1) Biomarker screening: Progenesis QI software was used to generate peak alignment, peak extraction, compound identification, and normalization on the raw data collected by mass spectrometry, and output feature information such as mass-to-charge ratio (m/z), retention time, and abundance. The *Acinetobacter baumannii* clones of different resistance profiles were categorized into different groups for principal component analysis, with parameters for screening biomarkers set as: fold change>10, VIP>1, p value<0.05, CV<30%. The following 102 biomarkers were screened out:

| No. | Compound | No. | Compound | No. | Compound | No. | Compound |
|---|---|---|---|---|---|---|---|
| CP1 | 0.67_885.3152m/z | CP27 | 2.68_672.5593m/z | CP53 | 2.88_1358.5844m/z | CP79 | 2.98_1055.0739m/z |
| CP2 | 0.80_247.3097m/z | CP28 | 2.70_1343.0987m/z | CP54 | 2.89_1108.1168m/z | CP80 | 2.98_1055.5707m/z |
| CP3 | 0.82_937.3576m/z | CP29 | 2.71_102.1264m/z | CP55 | 2.91_1011.8289m/z | CP81 | 2.98_1063.0585m/z |
| CP4 | 0.82_937.8613m/z | CP30 | 2.73_1058.9230m/z | CP56 | 2.91_1012.8270m/z | CP82 | 2.98_1066.0654m/z |
| CP5 | 0.86_892.2968m/z | CP31 | 2.73_1279.0598m/z | CP57 | 2.91_1371.6639m/z | CP83 | 2.98_1066.5628m/z |
| CP6 | 0.87_275.1338m/z | CP32 | 2.73_1389.6516m/z | CP58 | 2.91_1372.2017m/z | CP84 | 2.98_1074.0492m/z |
| CP7 | 0.87_574.2563m/z | CP33 | 2.74_1070.9482m/z | CP59 | 2.91_915.8016m/z | CP85 | 2.98_1077.0566m/z |
| CP8 | 0.87_645.1994m/z | CP34 | 2.74_1082.9834m/z | CP60 | 2.92_1187.1433m/z | CP86 | 2.98_1260.7389m/z |
| CP9 | 1.46_583.3177m/z | CP35 | 2.75_731.6471m/z | CP61 | 2.92_1206.1139m/z | CP87 | 2.98_1356.6824m/z |
| CP10 | 1.95_119.0333m/z | CP36 | 2.76_1092.0217m/z | CP62 | 2.92_1293.6244m/z | CP88 | 2.98_666.0361m/z |
| CP11 | 1.95_136.0604m/z | CP37 | 2.76_1098.9938m/z | CP63 | 2.92_1329.7157m/z | CP89 | 2.98_703.7160m/z |
| CP12 | 1.95_268.1029m/z | CP38 | 2.76_1132.0044m/z | CP64 | 2.92_1365.6693m/z | CP90 | 2.98_704.0466m/z |
| CP13 | 2.18_330.0594m/z | CP39 | 2.76_119.0118m/z | CP65 | 2.93_1269.6239m/z | CP91 | 2.98_709.0372m/z |
| CP14 | 2.18_393.1092m/z | CP40 | 2.76_1459.7094m/z | CP66 | 2.94_948.1554m/z | CP92 | 2.98_921.9998m/z |
| CP15 | 2.24_277.5730m/z | CP41 | 2.77_687.6290m/z | CP67 | 2.95_1342.7146m/z | CP93 | 2.98_998.0538m/z |
| CP16 | 2.35_242.5698m/z | CP42 | 2.78_1126.0419m/z | CP68 | 2.95_947.8219m/z | CP94 | 3.00_1036.5737m/z |
| CP17 | 2.39_98.0585m/z | CP43 | 2.78_1299.6447m/z | CP69 | 2.95_948.8201m/z | CP95 | 3.00_1093.1141m/z |
| CP18 | 2.41_507.5750m/z | CP44 | 2.78_1316.6255m/z | CP70 | 2.96_1257.2006m/z | CP96 | 3.00_1093.6160m/z |
| CP19 | 2.41_511.5846m/z | CP45 | 2.78_1338.6192m/z | CP71 | 2.97_1052.0905m/z | CP97 | 3.00_1094.1189m/z |
| CP20 | 2.42_492.0796m/z | CP46 | 2.78_1491.7467m/z | CP72 | 2.97_1421.7925m/z | CP98 | 3.00_1104.1054m/z |
| CP21 | 2.60_1411.5904m/z | CP47 | 2.85_1182.0766m/z | CP73 | 2.97_1443.7972m/z | CP99 | 3.00_1104.6071m/z |
| CP22 | 2.63_1133.9809m/z | CP48 | 2.85_1329.6451m/z | CP74 | 2.97_708.7048m/z | CP100 | 3.00_1115.0977m/z |
| CP23 | 2.65 908.9106m/z | CP49 | 2.85_1444.7037m/z | CP75 | 2.97_837.7936m/z | CP101 | 3.00_729.7483m/z |
| CP24 | 2.66_1421.5652m/z | CP50 | 2.86_1374.1612m/z | CP76 | 2.97_838.1319m/z | CP102 | 3.00_730.0794m/z |
| CP25 | 2.67_1250.6971m/z | CP51 | 2.86_1411.6575m/z | CP77 | 2.98_1009.0445m/z | | |
| CP26 | 2.67_1395.8226m/z | CP52 | 2.86 1471.7506m/z | CP78 | 2.98_1044.0853m/z | | |

(2) Resistance profile classification: According to the susceptibility properties of *Acinetobacter baumannii* to 12 antibacterial drugs including piperacillin, ceftazidime, cefepime, imipenem, meropenem, gentamicin, tobramycin, amikacin, levofloxacin, ciprofloxacin, TMP-SMZ and minocycline, its resistance profiles were classified into different types, named as A to S. The drug resistance profile classification and corresponding drug susceptibility are shown in Table 2.

**Table 2**

| Resistance profile classification | Susceptibility of *Acinetobacter baumannii* to 12 antibacterial drugs | | | | | | | | | | | |
|---|---|---|---|---|---|---|---|---|---|---|---|---|
| | piperacillin | ceftazidime | cefepime | imipenem | meropenem | gentamicin | tobramycin | amikacin | levofloxacin | ciprofloxacin | TMP-SMZ | minocycline |
| A | R | R | R | R | R | R | R | R | R | R | R | S |
| B | R | R | R | R | R | R | S | S | R | R | S | S |
| C | R | R | R | R | R | R | R | R | R | R | S | S |
| D | R | R | R | R | R | R | S | S | R | R | R | S |
| E | R | R | R | R | R | S | S | S | R | R | R | I |
| F | R | R | R | R | R | R | R | S | R | R | R | S |
| G | R | R | R | R | R | S | S | S | R | R | R | S |
| H | R | R | R | R | R | S | S | S | R | R | S | S |
| I | R | R | R | R | R | S | S | S | S | S | S | S |
| J | R | S | R | R | R | R | S | S | R | R | R | S |
| K | R | R | S | R | R | R | R | R | R | R | R | S |
| L | R | R | R | S | S | R | R | S | R | R | R | S |
| M | R | R | R | S | S | R | S | S | R | R | S | S |
| N | R | R | R | S | S | S | S | S | R | R | S | S |
| O | S | S | S | S | S | R | R | S | S | S | S | S |
| P | S | S | S | S | S | R | S | S | S | S | R | S |
| Q | S | S | S | S | S | S | S | S | R | R | S | S |
| R | S | S | S | S | S | S | S | S | R | R | R | S |
| S | S | S | S | S | S | S | S | S | S | S | S | S |

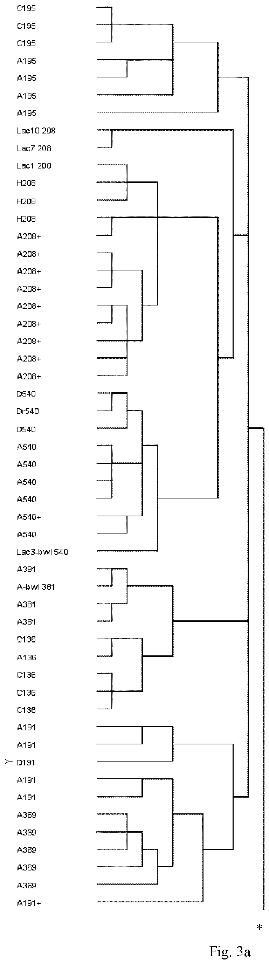

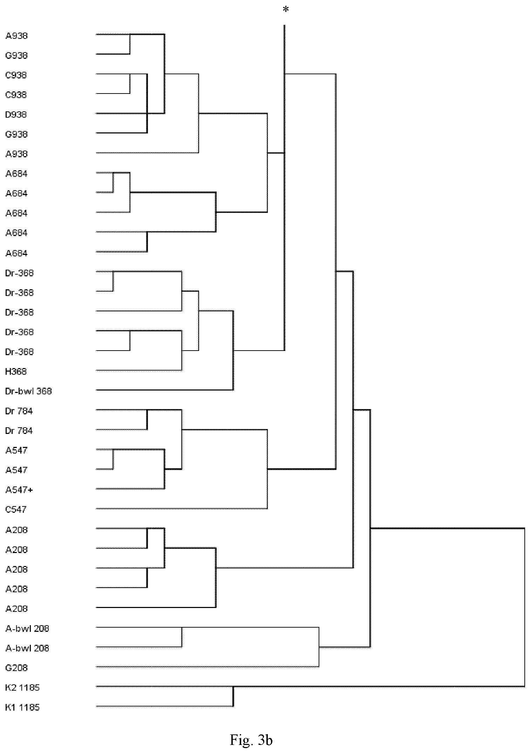

(3) Construction of a metabolic spectrum phylogenetic tree database for susceptibility determination: Through biomarker analysis, identical clones were merged and 87 representative *Acinetobacter baumannii* clones were selected for the database. The feature information of the biomarkers (retention time, mass-to-charge ratio, abundance) was imported into the software IBM SPSS Statistics 23, the retention time and mass-to-charge ratio information used as variable names, the compound abundance used as variable value. Using the analysis mode of 'systematic cluster', a dendrogram was generated, as shown in Figure 3.

### 3. Database validation and result interpretation

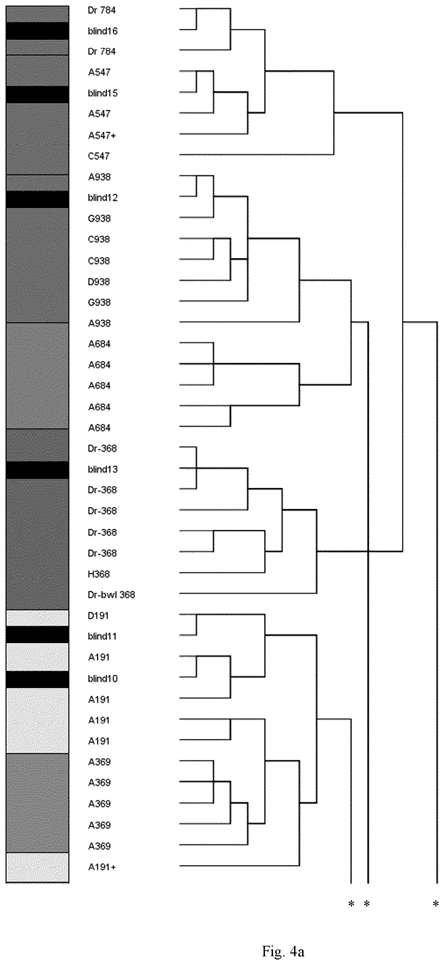

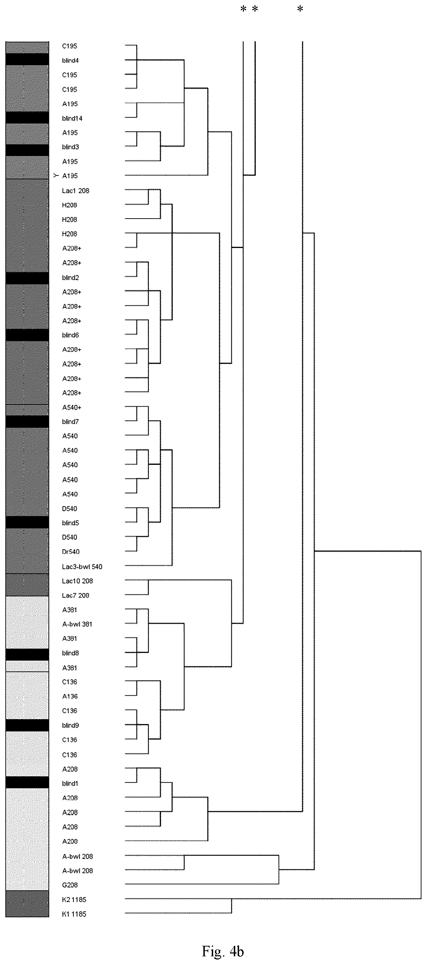

(1) Blind test: The metabolic fingerprints of 16 blinds were imported into the software IBM SPSS Statistics 23 for cluster analysis. The resistance profile of each sample was determined based on its positioning in the phylogenetic tree and the corresponding prediction rules. As is shown in Figure 4, the branches of the phylogenetic tree representing different resistance profiles are indicated by different colors on the left, where black represents blind samples.
(2) Result interpretation: The resistance profiles of 16 blinds were predicted based on their positioning in the phylogenetic tree and the corresponding prediction rules. Use independent prediction rules: when the test sample contains metabolic fingerprints of a specific clone, follow the principle that the drug resistance profile predicted by metabolic fingerprints is preferred over phylogenetic tree interpretation.

The resistance profiles of the 16 blinds to 12 antibacterial drugs including piperacillin, ceftazidime, cefepime, imipenem, meropenem, gentamicin, tobramycin, amikacin, levofloxacin, ciprofloxacin, TMP-SMZ and minocycline predicted by method of the present invention are shown in Table 3.

**Table 3**

| Sample | Resistance profiles by VITEK2 (the gold standard) | Resistance profiles inferred by phylogenetic tree | Prediction rules involved | Corrections | Agreement (preliminary result) | Agreement (final result) |
|---|---|---|---|---|---|---|
| bluid-1 | G | A208 | contains G208 biomarkers, preferred over phylogenetic tree | corrected to G208 | 1 False positive (gentamicin, tobramycin, amikacin) | yes |
| blind-2 | A | A208 | / | / | yes | yes |
| blind-3 | A | A195 | / | / | yes | yes |
| blind-4 | C | C195 | / | / | yes | yes |
| blind-5 | F | D540 | contains F540 biomarkers, preferred over phylogenetic tree | corrected to F540 | 1 False negative (tobramycin) | yes |
| blind-6 | A | A208 | / | / | yes | yes |
| blind- 7 | A | A540 | / | / | yes | yes |
| blind-8 | A | A381 | / | / | yes | yes |
| blind-9 | C | C136 | / | / | yes | yes |
| blind-10 | A | A191 | / | / | yes | yes |
| blind-11 | D | D191 | / | / | yes | yes |
| blind-12 | A | A938 | / | / | yes | yes |
| blind-13 | D | D368 | / | / | yes | yes |
| blind-14 | A | C195 | / | / | 1 False negative (TMP-SMZ) | 1 False negative (TMP-SMZ) |
| blind-15 | A | A547 | / | / | yes | yes |
| blind-16 | D | D784 | / | / | yes | yes |

When analyzed solely using the phylogenetic tree, out of 16 blinds, 3 samples and 6 antibiotics displayed inconsistent results compared with the gold standard VITEK2 AST. However, once the phylogenetic tree was combined with metabolic fingerprints and specific prediction rule was applied, the results were corrected. Since the blind 1 contained specific G208 biomarkers (2.41_507.5750m/z and 2.41_511.5846m/z) and the blind 5 contained specific F540 biomarkers (3.00_1093.6160m/z and 3.00_1094.1189m/z), the prediction rule that when the test sample contains metabolic fingerprints of a specific clone, the drug resistance profile predicted by metabolic fingerprints is preferred over phylogenetic tree interpretation was applied. Therefore, the results of blind sample 1 and blind sample 5 were revised to G208 and F540, which were in agreement with the gold standard VITEK2 AST.

Finally, in one case of the 16 blinds, the resistance profile predicted by method of the present invention was inconsistent with that of the gold standard VITEK2 AST, that is, the false negative result of TMP-SMZ. As for 12 drugs involved in this study, a total of 192 drug results were analyzed, displaying 1 false negative result and no false positive result. The positive predictive value was 100% (168/168), the negative predictive value was 95.83% (23/24), the sensitivity was 99.41% (168/169), and the specificity was 100% (23/23). The performances meet the design requirements of the present method and the needs of clinical application, that is, the sensitivity and specificity being above 95%.

### Example 3: Construction and validation of a metabolic spectrum phylogenetic tree database for Enterococcus faecalis based on LC-MS

*Enterococcus faecalis* was selected as the representative of Gram-positive cocci to illustrate the way a metabolic spectrum phylogenetic tree for drug susceptibility determination is constructed. Those skilled in the art can understand that this method is applicable to other Gram-negative bacteria including *Enterococcus faecium.* and the *Staphylococcus spp..*

### 1. Method

Step 1. Drug susceptibility verification: The *Enterococcus faecalis* isolates were tested for drug susceptibility using bioMérieux Vitek2 Compact System and AST-P639 cards, and the results were used as the gold standard (culture-based AST).

Step 2. Preparation of bacterial suspension: Each test isolate was inoculated on Columbia sheep blood agar plate, and after culturing overnight, an appropriate amount of colonies was scraped into a sterile saline tube with a disposable inoculation loop to prepare a homogeneous bacterial suspension.

Step 3. Cell breakage: an equal volume of bacterial standards was added to 180 µL of bacterial suspension, and sonicated at 80 Hz for 5 min.

Step 4. Extraction and concentration of metabolites: 340 µL of the sonication product obtained in step 3 was transferred to a 1.5 ml centrifuge tube, mixed with an equal volume of extraction buffer, shaked on ice for 3 min, and centrifuged quickly for 3-5 seconds. The liquid in the tube was spin down to the bottom of the tube and dried using a nitrogen blower.

Step 5. Mass spectrometry detection: the residue obtained in step 4 was resuspended with 140 µL of resuspension buffer, vortexed, centrifuged at high speed for 5 min. The supernatant was transferred to a new centrifuge tube, and centrifuged at high speed for 5 min. 100 µL of the supernatant was transferred to the sample introduction system of LC-MS, and 4 µL of the sample was injected each run for detection and analysis; preferably, the high-resolution LC-MS is Waters Q-TOF Synapt G2-Si quadrupole-time-of-flight mass spectrometer. The retention was achieved in gradient elution reversed-phase chromatography under the conditions as follows: water-acetonitrile-formic acid was used as the mobile phase system, the flow rate of the mobile phase was 0.4 ml/min, and the column temperature was 40 °C; the chromatographic column was a Waters HSS T3 column with a particle size of 1.8 µm, an inner diameter of 2.1 mm, and a column length of 100 mm; Mass spectrometry detection adopts electrospray ionization source (ESI), positive ion mode, multiple reaction monitoring scan mode (MRM) and MSeContinnum data independent acquisition mode.

### 2. Bioinformatics analysis and database construction

(1) Biomarker screening: Progenesis QI software was used to generate peak alignment, peak extraction, compound identification, and normalization on the raw data collected by mass spectrometry, and output feature information such as mass-to-charge ratio (m/z), retention time, and abundance. The *Enterococcus faecalis* clones of different resistance profiles were categorized into different groups for principal component analysis, with parameters for screening biomarkers set as: fold change>10, VIP>1, p value<0.05, CV<30%. The following 51 biomarkers were screened out:

| No. | Compound | No. | Compound | No. | Compound | No. | Compound |
|---|---|---|---|---|---|---|---|
| CP1 | 0.53_491.241 lm/z | CP14 | 1.04_347.1912m/z | CP27 | 2.50_697.3 3 39m/z | CP40 | 2.66_718.3410m/z |
| CP2 | 0.55_1409.6162m/z | CP15 | 1.06_192.5755m/z | CP28 | 2.51_867.4025m/z | CP41 | 2.66_724.3318m/z |
| CP3 | 0.55_1450.5881m/z | CP16 | 1.07_215.5787m/z | CP29 | 2.53_120.0864m/z | CP42 | 2.66_824.3855m/z |
| CP4 | 0.60_104.1128m/z | CP17 | 1.14_639.0793m/z | CP30 | 2.53_515.2600m/z | CP43 | 2.68_585.3086m/z |
| CP5 | 0.60_258.1144m/z | CP18 | 2.04_366.1352m/z | CP31 | 2.58_414.2292m/z | CP44 | 2.71_642.3346m/z |
| CP6 | 0.60_258.3054m/z | CP19 | 2.06_268.1078m/z | CP32 | 2.58_615.2853m/z | CP45 | 2.71_856.4256m/z |
| CP7 | 0.60_258.5580m/z | CP20 | 2.47_194.1219m/z | CP33 | 2.59_442.2250m/z | CP46 | 2.72_813.4196m/z |
| CP8 | 0.61_162.1183m/z | CP21 | 2.47_373.2003m/z | CP34 | 2.59_795.3734m/z | CP47 | 2.74_576.3378m/z |
| CP9 | 0.64_538.0832m/z | CP22 | 2.48_254.1650m/z | CP35 | 2.59_941.4294m/z | CP49 | 2.76_646.9948m/z |
| CP10 | 0.75_829.2745m/z | CP23 | 2.48_496.247 lm/z | CP36 | 2.61_610.3115m/z | CP50 | 2.76_684.3738m/z |
| CP11 | 0.79_991.3231m/z | CP24 | 2.48_764.3693m/z | CP37 | 2.61_757.3598m/z | CP51 | 2.84_659.3369m/z |
| CP12 | 0.97_664.1133m/z | CP25 | 2.49_516.0883m/z | CP38 | 2.63_711.3399m/z | | |
| CP13 | 1.02_308.0948m/z | CP26 | 2.50_343.1991m/z | CP39 | 2.66_188.0762m/z | | |

(2) Resistance profile classification: According to the susceptibility properties of *Enterococcus faecalis* to 11 antibacterial drugs including penicillin, ampicillin, vancomycin, linezolid, daptomycin, high-level gentamicin, erythromycin, levofloxacin, ciprofloxacin, tigecycline and tetracycline, its resistance profiles were classified into different types, named as A to S. The drug resistance profile classification and corresponding drug susceptibility are shown in Table 4.

**Table 4**

| Resistance profile classification | Susceptibility of *Enterococcus faecalis* to 11 antibacterial drugs | | | | | | | | | | |
|---|---|---|---|---|---|---|---|---|---|---|---|
| | penicillin | ampicillin | vancomycin | linezolid | daptomycin | HLAR | erythromycin | levofloxacin | ciprofloxacin | tigecycline | tetracycline |
| A | R | R | S | S | S | R | R | R | R | S | R |
| B | R | R | S | S | S | R | R | R | R | S | S |
| C | R | R | S | S | S | S | R | R | R | S | S |
| D | R | R | S | S | S | S | S | R | R | S | S |
| E | R | R | S | S | S | S | R | S | S | S | S |
| F | R | R | S | S | S | S | R | S | S | S | R |
| G | S | S | S | S | S | R | R | R | R | S | R |
| H | S | S | S | S | S | R | R | S | S | S | R |
| I | S | S | S | S | S | R | R | R | R | S | S |
| J | S | S | S | S | S | S | R | S | S | S | R |
| K | S | S | S | S | S | S | S | R | R | S | R |
| L | S | S | S | S | S | S | S | R | R | S | S |
| M | S | S | S | S | S | S | S | S | R | S | R |
| S | S | S | S | S | S | S | S | S | S | S | S |

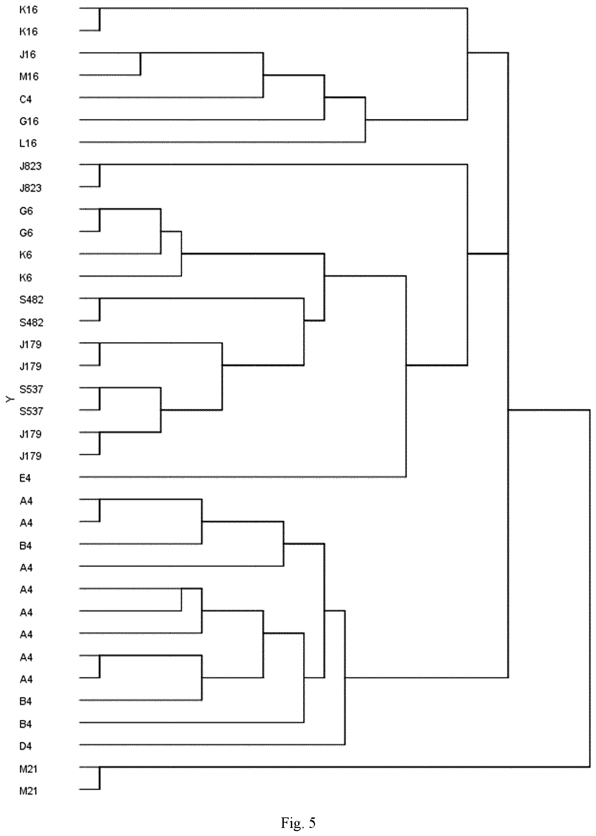

(3) Construction of a metabolic spectrum phylogenetic tree database for susceptibility determination: Through biomarker analysis, identical clones were merged and 36 representative *Enterococcus faecalis* clones were selected for the database. The feature information of the biomarkers (retention time, mass-to-charge ratio, abundance) was imported into the software IBM SPSS Statistics 23, the retention time and mass-to-charge ratio information used as variable names, the compound abundance used as variable value. Using the analysis mode of 'systematic cluster', a dendrogram was generated, as shown in Figure 5.

### 3. Database validation and result interpretation

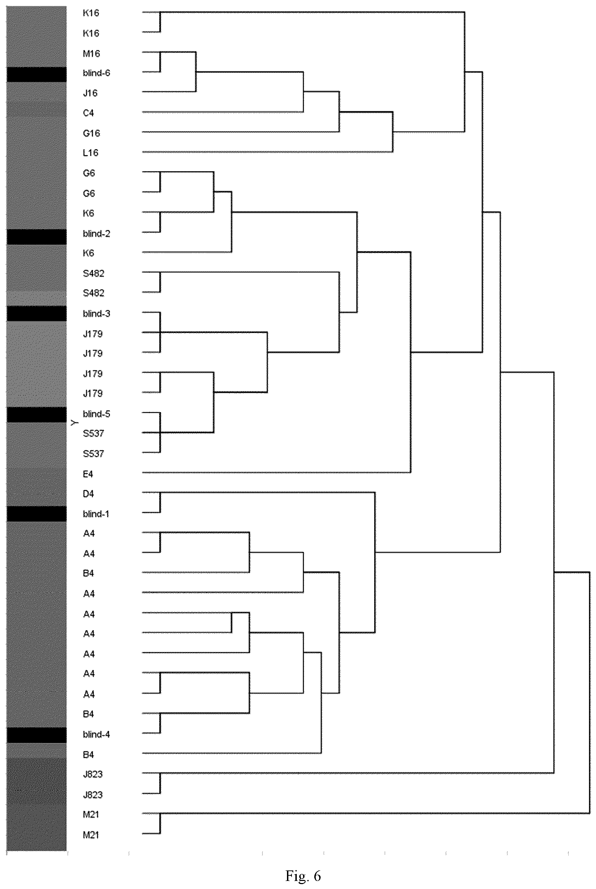

(1) Blind test: The metabolic fingerprints of 6 blinds were imported into the software IBM SPSS Statistics 23 for cluster analysis. The resistance profile of each sample was determined based on its positioning in the phylogenetic tree and the corresponding prediction rules. As is shown in Figure 6, the branches of the phylogenetic tree representing different resistance profiles are indicated by different colors on the left, where black represents blind samples.
(2) Result interpretation: The resistance profiles of 6 blinds were predicted based on their positioning in the phylogenetic tree and the corresponding prediction rules. Use independent prediction rules: a) when the test sample contains metabolic fingerprints of a specific clone, follow the principle that the drug resistance profile predicted by metabolic fingerprints is preferred over phylogenetic tree interpretation; b) when the test sample has the metabolic fingerprints of a sequence type 4 *Enterococcus faecalis* clone, the sample is directly determined to be resistant to penicillins.

The resistance profiles of the 6 blinds to 11 antibacterial drugs including penicillin, ampicillin, vancomycin, linezolid, daptomycin, high-level gentamicin, erythromycin, levofloxacin, ciprofloxacin, tigecycline and tetracycline predicted by method of the present invention are shown in Table 5.

**Table 5**

| Sample | Resistance profiles by VITEK2 (the gold standard) | Resistance profiles inferred by phylogenetic tree | Prediction rules involved | Corrections | Agreement (preliminary result) | Agreeme nt (final result) |
|---|---|---|---|---|---|---|
| blind-1 | D | D4 | / | / | yes | yes |
| blind-2 | K | K6 | / | / | yes | yes |
| blind-3 | J | J179 | / | / | yes | yes |
| blind-4 | B | B4 | / | / | yes | yes |
| blind-5 | S | S537 | / | / | yes | yes |
| blind-6 | A | M16 | contains ST4 biomarkers, preferred over phylogenetic tree | corrected to A4 | 1 False negative (penicillin, ampicillin, HLAR, erythromycin, levofloxacin, ciprofloxacin) | yes |

When analyzed solely using the phylogenetic tree, out of 6 blinds, 1 samples and 6 antibiotics displayed inconsistent results compared with the gold standard VITEK2 AST. However, once the phylogenetic tree was combined with metabolic fingerprints and specific prediction rule was applied, the results were corrected. Since the blind 6 contained specific ST4 biomarkers (1.06_192.5755m/z, 1.07_215.5787m/z, 1.14_639.0793m/z 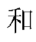 2.04_366.1352m/z), the prediction rule that when the test sample has the metabolic fingerprints of a sequence type 4 *Enterococcus faecalis* clone, the sample is directly determined to be resistant to penicillins was applied. Therefore, the result was revised to A4, which were in 100% agreement with the gold standard VITEK2 AST.

### Example 4: Construction and validation of a metabolic spectrum phylogenetic tree database for Streptococcus pneumoniae based on LC-MS

*Streptococcus pneumoniae* was selected as the representative of the fastidious bacteria to illustrate the way a metabolic spectrum phylogenetic tree for drug susceptibility determination is constructed.

### 1. Method

Step 1. Drug susceptibility verification: The *Streptococcus pneumoniae* isolates were tested for drug susceptibility using bioMérieux Vitek2 Compact System and AST-GP68 cards, and specifically, the susceptibility of penicillin was double checked by disc diffusion method (OXOID, CT0043B), and the combined results were used as the gold standard (culture-based AST).

Step 2. Preparation of bacterial suspension: Each test isolate was inoculated on Columbia sheep blood agar plate, and after culturing overnight, an appropriate amount of colonies was scraped into a sterile saline tube with a disposable inoculation loop to prepare a homogeneous bacterial suspension.

Step 3. Cell breakage: an equal volume of bacterial standards was added to 180 µL of bacterial suspension, and sonicated at 80 Hz for 5 min.

Step 4. Extraction and concentration of metabolites: 340 µL of the sonication product obtained in step 3 was transferred to a 1.5 ml centrifuge tube, mixed with an equal volume of extraction buffer, shaked on ice for 3 min, and centrifuged quickly for 3-5 seconds. The liquid in the tube was spin down to the bottom of the tube and dried using a nitrogen blower.

Step 5. Mass spectrometry detection: the residue obtained in step 4 was resuspended with 140 µL of resuspension buffer, vortexed, centrifuged at high speed for 5 min. The supernatant was transferred to a new centrifuge tube, and centrifuged at high speed for 5 min. 100 µL of the supernatant was transferred to the sample introduction system of LC-MS, and 4 µL of the sample was injected each run for detection and analysis; preferably, the high-resolution LC-MS is Waters Q-TOF Synapt G2-Si quadrupole-time-of-flight mass spectrometer. The retention was achieved in gradient elution reversed-phase chromatography under the conditions as follows: water-acetonitrile-formic acid was used as the mobile phase system, the flow rate of the mobile phase was 0.4 ml/min, and the column temperature was 40 °C; the chromatographic column was a Waters HSS T3 column with a particle size of 1.8 µm, an inner diameter of 2.1 mm, and a column length of 100 mm; Mass spectrometry detection adopts electrospray ionization source (ESI), positive ion mode, multiple reaction monitoring scan mode (MRM) and MSeContinnum data independent acquisition mode.

### 2. Bioinformatics analysis and database construction

(1) Biomarker screening: Progenesis QI software was used to generate peak alignment, peak extraction, compound identification, and normalization on the raw data collected by mass spectrometry, and output feature information such as mass-to-charge ratio (m/z), retention time, and abundance. The *Streptococcus pneumoniae* clones of different resistance profiles were categorized into different groups for principal component analysis, with parameters for screening biomarkers set as: fold change>10, VIP>1, p value<0.05, CV<30%. The following 21 biomarkers were screened out:

| No. | Compound | No. | Compound | No. | Compound | No. | Compound |
|---|---|---|---|---|---|---|---|
| CP1 | 0.66_382.0899m/z | CP7 | 1.16_366.0977m/z | CP13 | 2.99_1317.2267m/z | CP19 | 2.97_1134.1406m/z |
| CP2 | 0.71_1355.4229m/z | CP8 | 1.16_237.0537m/z | CP14 | 2.98_1305.7357m/z | CP20 | 2.96_1275.6738m/z |
| CP3 | 0.72_166.0547m/z | CP9 | 1.16_404.0425m/z | CP15 | 2.98_1306.2387m/z | CP21 | 2.26_1297.4318m/z |
| CP4 | 0.72_295.0939m/z | CP10 | 1.16_219.0439m/z | CP16 | 2.99_871.1594m/z | | |
| CP5 | 0.85_1487.5082m/z | CP11 | 1.78_443.7582m/z | CP17 | 2.99_870.826lm/z | | |
| CP6 | 1.16_202.5253m/z | CP12 | 2.99_884.1424m/z | CP18 | 2.99_878.8190m/z | | |

(2) Resistance profile classification: According to the susceptibility properties of *Streptococcus pneumoniae* to 14 antibacterial drugs including penicillin, amoxicillin, cefepime, cefotaxime, ceftriaxone, ertapenem, meropenem, erythromycin, TMP-SMZ, levofloxacin, moxifloxacin, vancomycin, linezolid and tetracycline, its resistance profiles were classified into different types, named as A to S. The drug resistance profile classification and corresponding drug susceptibility are shown in Table 6.

**Table 6**

| Resistance profile classification | Susceptibility of Streptococcus pneumoniae to 14 antibacterial drugs | | | | | | | | | | | | | |
|---|---|---|---|---|---|---|---|---|---|---|---|---|---|---|
| | penicillin | amoxicillin | cefepime | cefotaxime | ceftriaxone | ertapenem | meropenem | erythromycin | TMP-SMZ | levofloxacin | moxifloxacin | vancomycin | linezolid | tetracycline |
| A | R | R | R | R | R | S | S | R | R | S | S | S | S | R |
| B | R | R | R | R | R | S | S | S | R | S | S | S | S | R |
| C | R | R | R | R | R | S | S | R | R | S | S | S | S | S |
| D | R | R | R | R | R | S | S | S | R | S | S | S | S | S |
| E | R | R | R | R | R | S | S | R | S | S | S | S | S | S |
| F | R | R | R | R | R | S | S | S | S | S | S | S | S | S |
| G | S | S | S | S | S | S | S | R | R | S | S | S | S | R |
| H | S | S | S | S | S | S | S | R | S | S | S | S | S | R |
| I | S | S | S | S | S | S | S | R | S | S | S | S | S | S |
| J | S | S | S | S | S | S | S | S | R | S | S | S | S | R |
| S | S | S | S | S | S | S | S | S | S | S | S | S | S | S |

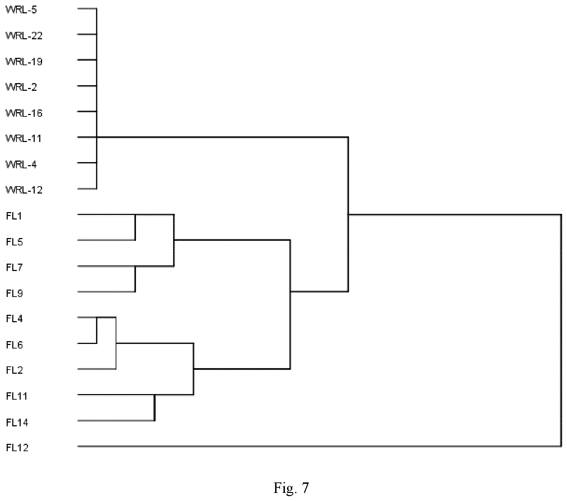

(3) Construction of a metabolic spectrum phylogenetic tree database for susceptibility determination: Through biomarker analysis, identical clones were merged and 18 representative *Streptococcus pneumoniae* clones were selected for the database. The feature information of the biomarkers (retention time, mass-to-charge ratio, abundance) was imported into the software IBM SPSS Statistics 23, the retention time and mass-to-charge ratio information used as variable names, the compound abundance used as variable value. Using the analysis mode of 'systematic cluster', a dendrogram was generated, as shown in Figure 7.

### 3. Database validation and result interpretation

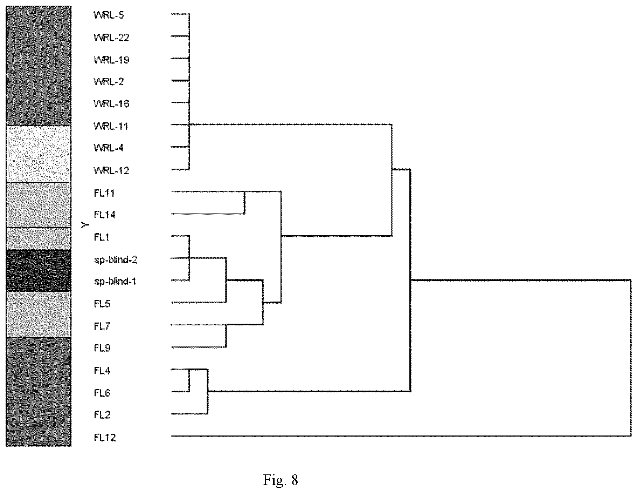

(1) Blind test: The metabolic fingerprints of 2 blinds were imported into the software IBM SPSS Statistics 23 for cluster analysis. The resistance profile of each sample was determined based on its positioning in the phylogenetic tree and the corresponding prediction rules. As is shown in Figure 8, the branches of the phylogenetic tree representing different resistance profiles are indicated by different colors on the left, where black represents blind samples.
(2) Result interpretation: The resistance profiles of 2 blinds were predicted based on their positioning in the phylogenetic tree and the corresponding prediction rules. Use independent prediction rules: a) when the test sample contains metabolic fingerprints of a specific clone, follow the principle that the drug resistance profile predicted by metabolic fingerprints is preferred over phylogenetic tree interpretation; b) when the test sample has the metabolic fingerprints of a *Streptococcus pneumoniae* clone with altered penicillin-binding protein patterns, the sample is directly determined to be resistant to penicillins and cephalosporinase.

The resistance profiles of the 2 blinds to 14 antibacterial drugs including penicillin, amoxicillin, cefepime, cefotaxime, ceftriaxone, ertapenem, meropenem, erythromycin, TMP-SMZ, levofloxacin, moxifloxacin, vancomycin, linezolid and tetracycline predicted by method of the present invention are shown in Table 7.

**Table 7**

| Sample | Resistance profiles by VITEK2 (the gold standard) | Resistance profiles inferred by phylogenetic tree | Prediction rules involved | Correctio ns | Agreement (preliminary result) | Agreeme nt (final result) |
|---|---|---|---|---|---|---|
| blind-1 | G | A | contains penicillin biomarkers, preferred over phylogenetic tree | corrected to G | 5 False negative (penicillins, cephalosporinase) | yes |
| blind-2 | A | A | / | / | yes | yes |

When analyzed solely using the phylogenetic tree, out of 2 blinds, 1 samples and 5 antibiotics displayed inconsistent results compared with the gold standard VITEK2 AST. However, once the phylogenetic tree was combined with metabolic fingerprints and specific prediction rule was applied, the results were corrected. Since the blind 1 has metabolic fingerprints of a *Streptococcus pneumoniae* clone with altered penicillin-binding protein patterns, the prediction rule that when the test sample has the metabolic fingerprints of a *Streptococcus pneumoniae* clone with altered penicillin-binding protein patterns, the sample is directly determined to be resistant to penicillins and cephalosporinase was applied. Therefore, the result was revised to G, which were in 100% agreement with the gold standard VITEK2 AST.

### Example 5: Construction and validation of a metabolic spectrum phylogenetic tree database for fungal identification based on LC-MS

### 1. Method

Step 1. Sample collection and identification: 420 clinical isolates were collected from 31 hospitals across china in a period between September 2015 and January 2019. All isolates were subjected to Sanger sequencing, as the gold standard for species identification.

Step 2. Preparation of bacterial suspension: Each test isolate was inoculated on Chromogenic agar plate, and after culturing overnight, an appropriate amount of colonies was scraped into a sterile saline tube with a disposable inoculation loop to prepare a homogeneous fungal suspension.

Step 3. Cell breakage: An equal volume of fungal standards was added to 180 µL of fungal suspension, and sonicated at 80 Hz for 5 min.

Step 4. Extraction and concentration of metabolites: 340 µL of the sonication product obtained in step 3 was transferred to a 1.5 ml centrifuge tube, mixed with an equal volume of extraction buffer, shaked on ice for 3 min, and centrifuged quickly for 3-5 seconds. The liquid in the tube was spin down to the bottom of the tube and dried using a nitrogen blower.

Step 5. Mass spectrometry detection: The residue obtained in step 4 was resuspended with 140 µL of resuspension buffer, vortexed, centrifuged at high speed for 5 min. The supernatant was transferred to a new centrifuge tube, and centrifuged at high speed for 5 min. 100 µL of the supernatant was transferred to the sample introduction system of LC-MS, and 4 µL of the sample was injected each run for detection and analysis; preferably, the high-resolution LC-MS is Waters Q-TOF Synapt G2-Si quadrupole-time-of-flight mass spectrometer. The retention was achieved in gradient elution reversed-phase chromatography under the conditions as follows: water-acetonitrile-formic acid was used as the mobile phase system, the flow rate of the mobile phase was 0.4 ml/min, and the column temperature was 40 °C; the chromatographic column was a Waters HSS T3 column with a particle size of 1.8 µm, an inner diameter of 2.1 mm, and a column length of 100 mm; Mass spectrometry detection adopts electrospray ionization source (ESI), positive ion mode, multiple reaction monitoring scan mode (MRM) and MSeContinnum data independent acquisition mode.

### 2. Bioinformatics analysis and database construction

(1) Biomarker screening: Progenesis QI software was used to generate peak alignment, peak extraction, compound identification, and normalization on the raw data collected by mass spectrometry, and output feature information such as mass-to-charge ratio (m/z), retention time, and abundance. Fungi of different species were categorized into different groups for principal component analysis, with parameters for screening biomarkers set as: fold change>10, VIP>1, p value<0.05, CV<30%. The following 72 biomarkers were screened out:

| | | | |
|---|---|---|---|
| 0.53_312.6547m/z | 0.53_455.2273m/z | 0.53_462. 7313m/z | 0.53_601. 3308m/z |
| 0. 53_639. 3055m/z | 0.53_651. 3450m/z | 0.53_675. 3021m/z | 0.53_783.4167m/z |
| 0.53_797.4358m/z | 0.55_1266.5582m/z | 0.55_1284.5517m/z | 0.55_1331.5422m/z |
| 0.62_162.2207m/z | 0.62 162.2590m/z | 0.62_162.4624m/z | 0.62 187.5477m/z |
| 0.65_148.0955m/z | 0.68 161.9788m/z | 0.70_713. 3483m/z | 0.70_754. 3701m/z |
| 0.72_158. 5687m/z | 0.72_190. 1422m/z | 0.75_258. 3427m/z | 0.80_1315.4491m/z |
| 0.88_1135.3840m/z | 0.95 195.5753m/z | 1.00_1354.3790m/z | 1.02_371.0143m/z |
| 1. 02_599. 0594m/z | 2.25_472. 9171m/z | 2.44_611. 9771m/z | 2.46_254. 2982m/z |
| 2.46_531.9382m/z | 2.46_588.6445m/z | 2.46_598.6573m/z | 2.46_882.9598m/z |
| 2.48_102.0899m/z | 2.48_407. 5446m/z | 2.49_318. 5222m/z | 2.49_318.8541m/z |
| 2. 49_391. 9494m/z | 2.49_734.0538m/z | 2.50_417. 9738m/z | 2.54_643. 9956m/z |
| 2. 54_647. 5503m/z | 2.56_1035. 1450m/z | 2.56_487. 0010m/z | 2.56_643. 5541m/z |
| 2.56_776.6147m/z | 2.58_530.0289m/z | 2.58_599.0957m/z | 2.58_599.4973m/z |
| 2. 58_698. 5924m/z | 2.59_1032. 1849m/z | 2.59_758.0302m/z | 2.60_453. 5753m/z |
| 2. 63_1173. 2365m/z | 2.63_704. 1436m/z | 2.64_687. 0079m/z | 2.65 656. 9775m/z |
| 2.66_847.6572m/z | 2.75_912.0944m/z | 2.78_774. 1840m/z | 2.79_541.6204m/z |
| 2.81_780. 7309m/z | 2.82_261.0359m/z | 2.82_305.0075m/z | 2.82_307.0062m/z |
| 2.83_1014. 1838m/z | 2.44 569. 9670m/z | 2.44_853. 9486m/z | 0.62_162. 1116m/z |

(2) Construction of a metabolic spectrum phylogenetic tree database: To optimize the resolution of the database, closely-related species such as *Candida parapsilosis, Candida orthopsilosis* and *Candida metapsilosis* which could not be accurately distinguished by the automated identification system such as VITEK2, were selected and added to the database.

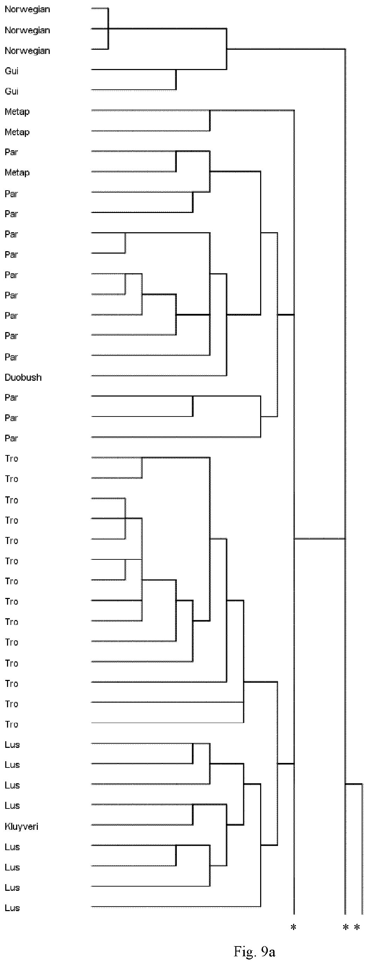

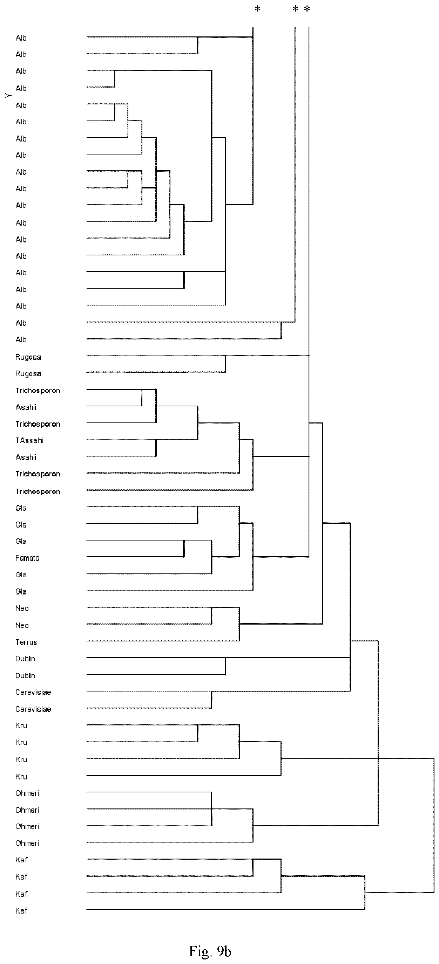

Through biomarker analysis, identical clones were merged and 115 representative clones were selected for the database. The feature information of the biomarkers (retention time, mass-to-charge ratio, abundance) was imported into the software IBM SPSS Statistics 23, the retention time and mass-to-charge ratio information used as variable names, the compound abundance used as variable value. Using the analysis mode of 'systematic cluster', a dendrogram was generated, as shown in Figure 9.

### 3. Database validation and result interpretation

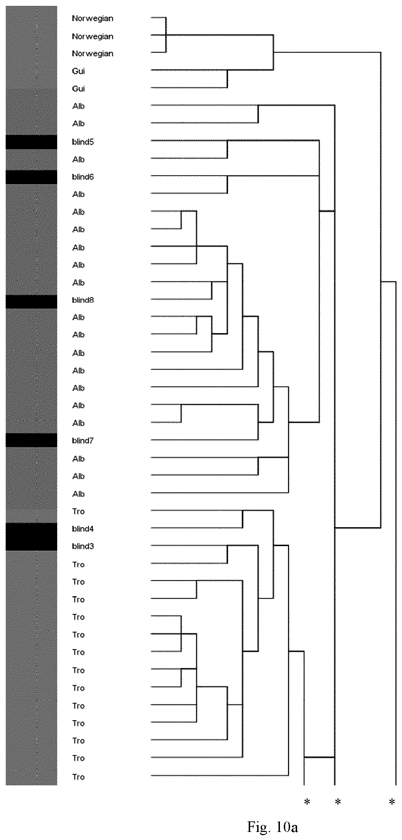

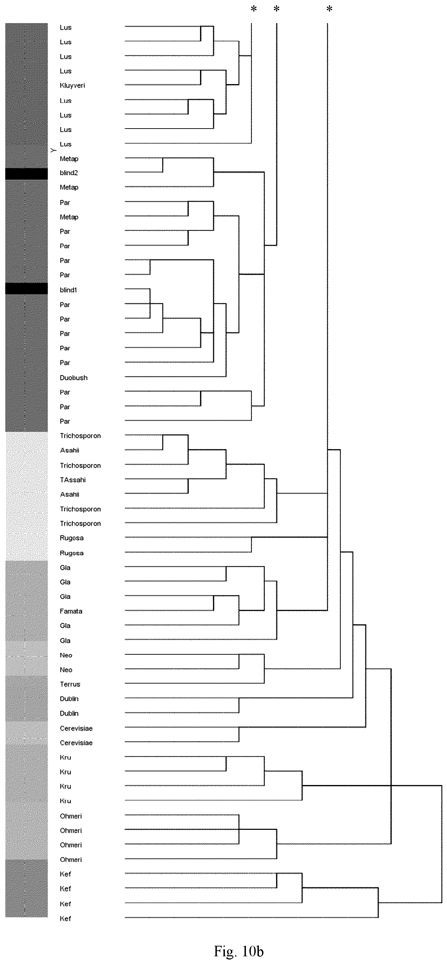

(1) Blind test: The metabolic fingerprints of 8 blinds were imported into the software IBM SPSS Statistics 23 for cluster analysis. The species of each blind was determined based on its positioning in the phylogenetic tree. As is shown in Figure 10, the branches of the phylogenetic tree representing different species are indicated by different colors on the left, where black represents blind samples.
(2) Result interpretation: The species of 8 blinds were predicted based on their positioning in the phylogenetic tree and the corresponding prediction rules. As is shown in Table 8, identification results predicted by method of this invention were in a 100% agreement with the gold standard (sequencing method) result. Notably, closely relates species such as *Candida parapsilosis* and *Candida metapsilosis,* which can not be distinguished by automated identification systems such as VITEK2, were identified in complete agreement with the gold standard method.

**Table 8**

| Sample | result by method of this invention | result by gold standard (sequencing) | agreement |
|---|---|---|---|
| blind-1 | *Candida parapsilosis* | *Candida parapsilosis* | yes |
| blind-2 | *Candida metapsilosis* | *Candida metapsilosis* | yes |
| blind-3 | *Candida tropicalis* | *Candida tropicalis* | yes |
| blind-4 | *Candida tropicalis* | *Candida tropicalis* | yes |
| blind-5 | *Candida albicans* | *Candida albicans* | yes |
| blind-6 | *Candida albicans* | *Candida albicans* | yes |
| blind-7 | *Candida albicans* | *Candida albicans* | yes |
| blind-8 | *Candida albicans* | *Candida albicans* | yes |

The prediction results of the 8 blind samples based on the phylogenetic tree positioning analysis of the metabolic profile database were in 100% agreement with those of the gold standard (sequencing method). Particularly, the results of blind 1 and blind 2 revealed that the resolution of the present method for fungal identification was at the subspecies level.

### Example 6: Construction and validation of a metabolic spectrum phylogenetic tree database for Candida tropicalis based on LC-MS

*Candida tropicalis* is the second most common pathogen of the invasive fungal infection after *Candida albicans,* and its triazole resistance rate is much higher than that of *Candida albicans* (30% vs 5%), and thus the prediction of resistance is more valuable in *Candida tropicalis.* Therefore, *Candida tropicalis* was selected as the representative of fungi to illustrate the way a metabolic spectrum phylogenetic tree for drug susceptibility determination is constructed. Those skilled in the art can understand that this method is applicable to other *Candida spp.* Or other yeasts.

### 1. Method

Step 1. Drug susceptibility verification: The *Candida tropicalis* isolates were tested for drug susceptibility using broth microdilution method following the M27-A3 and M27-S4 guidelines of NCCLS, and the results were used as the the gold standard (culture-based AST).

Step 2. Preparation of bacterial suspension: Each test isolate was inoculated on Chromogenic agar plate, and after culturing overnight, an appropriate amount of colonies was scraped into a sterile saline tube with a disposable inoculation loop to prepare a homogeneous fungal suspension.

Step 3. Cell breakage: an equal volume of fungal standards was added to 180 µL of bacterial suspension, and sonicated at 80 Hz for 5 min.

Step 4. Extraction and concentration of metabolites: 340 µL of the sonication product obtained in step 3 was transferred to a 1.5 ml centrifuge tube, mixed with an equal volume of extraction buffer, shaked on ice for 3 min, and centrifuged quickly for 3-5 seconds. The liquid in the tube was spin down to the bottom of the tube and dried using a nitrogen blower.

Step 5. Mass spectrometry detection: the residue obtained in step 4 was resuspended with 140 µL of resuspension buffer, vortexed, centrifuged at high speed for 5 min. The supernatant was transferred to a new centrifuge tube, and centrifuged at high speed for 5 min. 100 µL of the supernatant was transferred to the sample introduction system of LC-MS, and 4 µL of the sample was injected each run for detection and analysis; preferably, the high-resolution LC-MS is Waters Q-TOF Synapt G2-Si quadrupole-time-of-flight mass spectrometer. The retention was achieved in gradient elution reversed-phase chromatography under the conditions as follows: water-acetonitrile-formic acid was used as the mobile phase system, the flow rate of the mobile phase was 0.4 ml/min, and the column temperature was 40 °C; the chromatographic column was a Waters HSS T3 column with a particle size of 1.8 µm, an inner diameter of 2.1 mm, and a column length of 100 mm; Mass spectrometry detection adopts electrospray ionization source (ESI), positive ion mode, multiple reaction monitoring scan mode (MRM) and MSeContinnum data independent acquisition mode.

### 2. Bioinformatics analysis and database construction

(1) Biomarker screening: Progenesis QI software was used to generate peak alignment, peak extraction, compound identification, and normalization on the raw data collected by mass spectrometry, and output feature information such as mass-to-charge ratio (m/z), retention time, and abundance. The *Enterococcus faecalis* clones of different resistance profiles were categorized into different groups for principal component analysis, with parameters for screening biomarkers set as: fold change>10, VIP>1, p value<0.05, CV<30%. The following 22 biomarkers were screened out:

| No. | Compound | No. | Compound | No. | Compound | No. | Compound |
|---|---|---|---|---|---|---|---|
| CP1 | 0.88_1135.3840m/z | CP7 | 2.44 611.9771m/z | CP13 | 2.48 102.0899m/z | CP19 | 2.50_417.9738m/z |
| CP2 | 0.95_195.5753m/z | CP8 | 2.46_254.2982m/z | CP14 | 2.48_407.5446m/z | CP20 | 2.54_643.9956m/z |
| CP3 | 1.00_1354.3790m/z | CP9 | 2.46_531.9382m/z | CP15 | 2.49_318.5222m/z | CP21 | 2.54_647.5503m/z |
| CP4 | 1.02_371.0143m/z | CP10 | 2.46_588.6445m/z | CP16 | 2.49_318.8541m/z | CP22 | 2.56_1035.1450m/z |
| CP5 | 1.02_599.0594m/z | CP11 | 2.46_598.6573m/z | CP17 | 2.49_391.9494m/z | | |
| CP6 | 2.25_472.9171m/z | CP12 | 2.46_882.9598m/z | CP18 | 2.49_734.0538m/z | | |

(2) Resistance profile classification: Since 5-flucytosine, amphotericin B and echinocandin-resistance is rarely observed in clinical isolates of *Candida tropicalis,* its resistant profiles are categorized into two types: azole-resistant (pan-resistant to triazoles, e.g., fluconazole, itraconazole, voriconazole) and azole-susceptible.
(3) Construction of a metabolic spectrum phylogenetic tree database for susceptibility determination: Through biomarker analysis, identical clones were merged and 60 representative *Candida tropicalis* clones were selected for the database, among which 27 were azole-resistant and 33 were azole- susceptible. The feature information of the biomarkers (retention time, mass-to-charge ratio, abundance) was imported into the software IBM SPSS Statistics 23, the retention time and mass-to-charge ratio information used as variable names, the compound abundance used as variable value. Using the analysis mode of 'systematic cluster', a dendrogram was generated.

### 3. Database validation and result interpretation

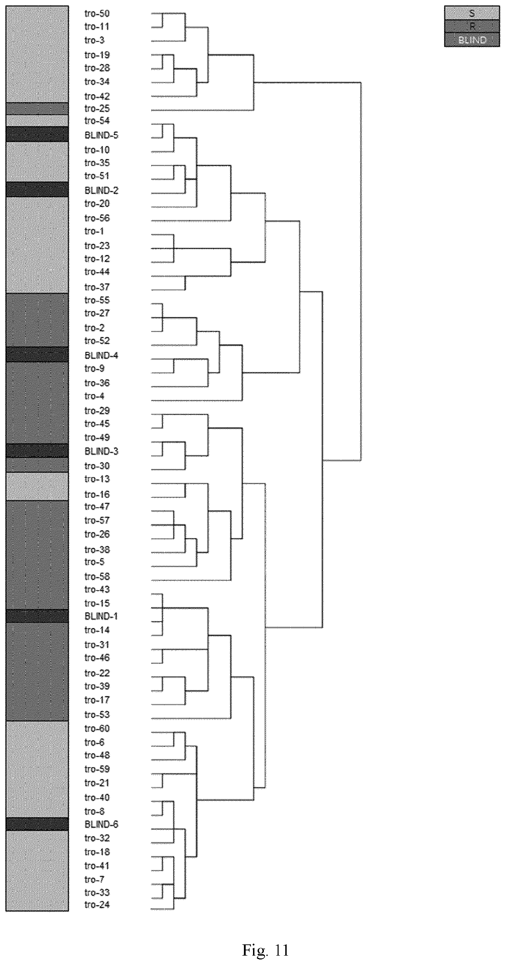

(1) Blind test: The metabolic fingerprints of 6 blinds were imported into the software IBM SPSS Statistics 23 for cluster analysis. The species of each blind was determined based on its positioning in the phylogenetic tree. As is shown in Figure 11, the branches of the phylogenetic tree representing different resistance profiles are indicated by different colors on the left, where blue indicates azole-susceptible, yellow indicates azole-resistant, and black represents blind samples.
(2) Result interpretation: The resistance profiles of 6 blinds were predicted based on their positioning in the phylogenetic tree and the corresponding prediction rules. Use independent prediction rules: strictly follow the principle that the drug resistance profile of a fungal strain is inferred on the basis of its closest relatives in the metabolic spectrum phylogenetic tree.

The resistance profiles of the 6 blinds to 6 antifungal drugs including 5-flucytosine, amphotericin B, fluconazole, itraconazole, voriconazole and caspofungin predicted by method of the present invention are shown in Table 9.

**Table 9**

| | branch location | Resistance profiles inferred by phylogenetic tree | Resistance profiles by broth microdilution (the gold standard) | | | | | | Agreeme nt |
|---|---|---|---|---|---|---|---|---|---|
| | | | 5-FC | AMB | FLU | ITR | VOR | CAS | |
| blind-1 | yellow | pan-azole resistant | S | S | R | R | R | S | yes |
| blind-2 | blue | susceptible to all drugs | S | S | S | S | S | S | yes |
| blind-3 | yellow | pan-azole resistant | S | S | R | R | R | S | yes |
| blind-4 | yellow | pan-azole resistant | S | S | R | R | R | S | yes |
| blind-5 | blue | susceptible to all drugs | S | S | S | S | S | S | yes |
| blind-6 | blue | susceptible to all drugs | S | S | S | S | S | S | yes |

The prediction results of the 6 blind samples based on the phylogenetic tree positioning analysis of the metabolic profile database were in 100% agreement with those of broth microdilution (the gold standard).

### Example 7: Construction and validation of a genomic phylogenetic tree database for Klebsiella pneumoniae based on WGS

*Klebsiella pneumoniae* was selected as the representative of Enterobacteriaceae to illustrate the way a genomic phylogenetic tree for drug susceptibility determination is constructed. Other Enterobacteriaceae can refer to this method for library construction and analysis.

### 1. Method

Step 1. Sample collection and drug susceptibility verification: 240 clinical *Klebsiella pneumoniae* isolates were collected from 23 hospitals across china in a period between January 2018 and March 2019. All isolates were tested for drug susceptibility using bioMérieux Vitek2 Compact System and AST-GN13/GN334 cards, and specifically, and the esults were used as the gold standard (culture-based AST).

Step 2. Genomic DNA preparation: Strains were inoculated on Columbia sheep blood agar plates by streaking and placed in an incubator (37°C) for 24 hours. The bacterial precipitate was then collected by centrifugation at 10,000 rpm for 2 mins, and the genomic DNA was purified from the pellets using DNeasy Blood and Tissue kit following the protocol by the manufacturer. DNA concentration was measured using Qubit fluorometer and the QC was performed on a NanoDropTM spectrophotometer, DNA with OD 260/280 1.6-2.0 and 260/230 2.0-2.2 were accepted for nanopore sequencing library preparation.

Step 3. Library preparation: ONT Native Barcoding Kit ID (EXP-NBD104 & 114) and Ligation Sequencing Kit ID (SQK LSK109) were used in library preparation following the ID Native barcoding genomic DNA protocol developed by ONT with a few modifications. The procedure is briefly described below, DNA extracted from each isolate were quantified on a Qubit 3.0 fluorometer and diluted to 20ng/µ1 with nuclease-free water (NF water), 50µl diluted DNA was used as starting material and incubated with 7µl Ultra II End-Prep reaction buffer and 3µl Ultra II End-Prep enzyme mix (New England Biolabs, USA) for 5 minutes at 20°C follow by 5 minutes at 65°C. The end-prepped DNA was then purified from the reaction mix using 1 × (v/v) AMPure XP magnetic beads and eluted with 25µl NF water. After quantification, elution containing 500ng DNA was obtained from each sample and topped up to 22.5µl with NF water and mixed with 2.5µl unique Native Barcode and 25µl Blunt/TA Ligation Master Mix. The mixture was incubated at room temperature for 10min and purified with 1× (v/v) AMPure XP magnetic beads, and the barcoded DNA was eluted with 26µl NF water. In the final step, equal amounts of 12 individually barcoded DNA samples were pooled to 700ng in total, and extra NF water were added to 50µl final volume. To the pooled DNA, 20µl ONT Barcode Adapter Mix, 20µl NEBNext Quick Ligation Reaction Buffer (5X) and 10µl Quick T4 DNA Ligase was added in order and mixed thoroughly, and after 10 minutes incubation at room temperature, the BAM ligated DNA was purified from the reaction mix with 0.4× (v/v) AMPure XP magnetic beads, and eluted in 15µl NF water.

Step 4. Nanopore sequencing: The sequencing consumable used in this study was ONT flowcell FLO-MIN106 R9.4. After flowcell priming, 75µL prepared library (35µL Running buffer, 255µL loading beads, and 14.5µL pooled library) was loaded. Sequencing was performed on an ONT MinIONTM portable sequencing device, and set and monitored using ONT MinKNOWTM desk software. Samples were pooled and sequenced for about 6 hours or until 1Gb of data for each sample were generated for database isolates and clinical specimens, respectively.

Step 5. Bioinformatics analysis: Raw FAST5 reads files were base-called using the Guppy v3.2.4 basecalling software. And the CANU 2.0 software was used to assemble the reads in fastq files into genomic assemblies or contigs with default parameters. The assembled genomes were analyzed using the local antibiotic resistance database for resistance determinants identification. Called fastq files of clinical specimens were analyzed with the EPI2ME WIMP rev.3.3.1 pipeline for the identification of pathogens present in the sample and reads classified as A. baumannii were extracted for further resistance analysis. A local nBLAST tool was created for interrogating contigs of clinical samples for a panel of validated AMR determinants. Phylogenetic analysis was conducted with kSNP3 (version 3.1) based on pan-genome SNPs identified and a Kmer value of 31 was adopted

### 2. Database construction and analytical logic establishment

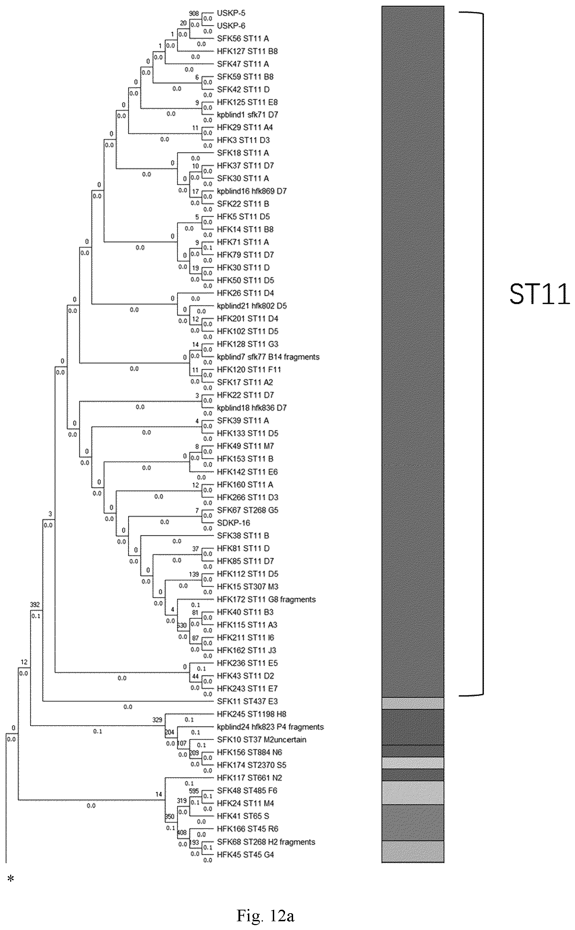

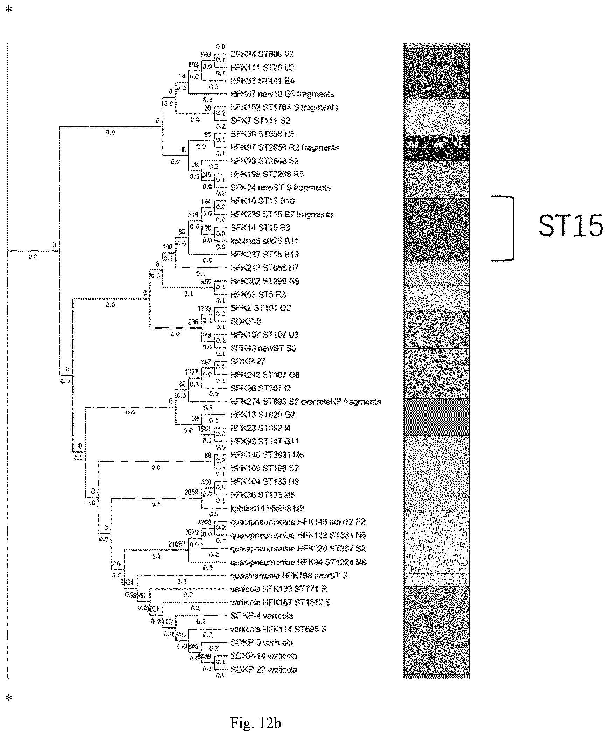

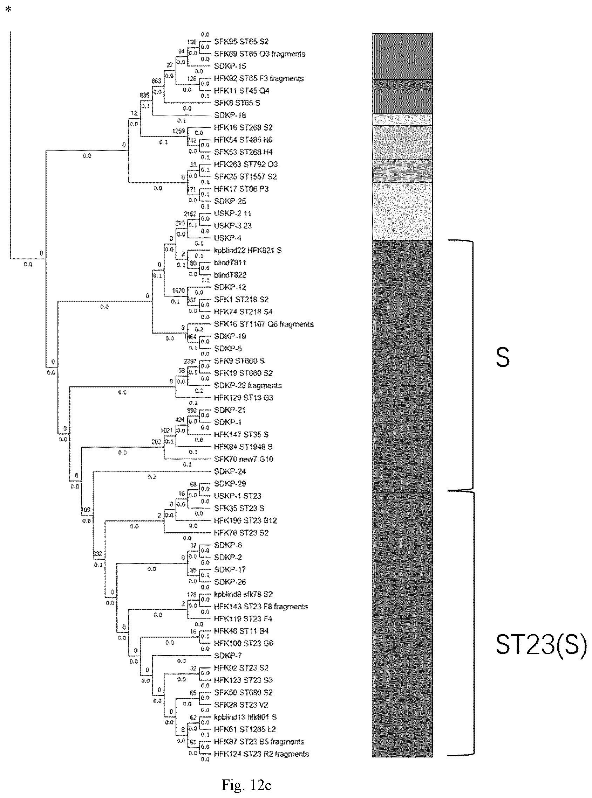

(1) Construction of a genomic phylogenetic tree for susceptibility determination: Through biomarker analysis, identical clones were merged and 165 representative *Klebsiella pneumonia* clones were selected for the database and a genomic phylogenetic tree database was built as shown in Figure 12. The branches of the phylogenetic tree representing different resistance profiles are indicated by different colors on the right, where yellow indicates ST11 clone, red indicates ST15 clone, and blue indicates susceptible to all antibiotics (including ST23 clone).
(2) Analytical logic establishment
(2a) Species identification: When the genome size of the test sample conforms to the range of 5,200,000-5,600,000bp, fragments of different lengths from multiple sites in the genome were selected and blasted against the NCBI nucleotide database. The species of the pathogen is determined to be *Klebsiella pneumoniae* only when the strain description shows *Klebsiella pneumoniae* and the per identity value exceeds 98%.
(2b) Drug susceptibility determination: When the test sample is located in the ST11 cluster (the yellow branch in Figure 12) of the genomic phylogenetic tree, its resistance profiles to 23 antibacterial drugs including ampicillin-sulbactam, piperacillin-tazobactam, cefoperazone-sulbactam, amoxicillin-clavulanate, cefazolin, cefuroxime, cefotaxime, ceftriaxone, ceftazidime, cefepime, aztreonam, cefoxitin, cefotetan, meropenem, ertapenem, imipenem, gentamicin, tobramycin, amikacin, ciprofloxacin, levofloxacin, tetracycline and TMP-SMZ is inferred according to the following ST11-type interpretation rules, that is, the drug resistance profile predicted by phylogenetic tree interpretation is preferred over antimicrobial resistance determinants analysis, and the test sample is directly determined to be resistant to cephalosporins, cephamycins, carbapenems and quinolones. The drug species and antimicrobial resistance determinants used in this study are shown in Table 10.

**Table 10**

| Antibiotics | Resistance profiles inferred by phylogenetic tree | Antimicrobial resistance determinants (partial) | | | | |
|---|---|---|---|---|---|---|
| | | *rmtB* | *aac(6')-I b3* | *aph(3')-I a* | *sull*/*2*/*3* | *tet(A)* |
| Ampicillin-sulbactam | R | | | | | |
| Piperacillin-tazobactam | R | | | | | |
| Cefoperazone-sulbactam | R | | | | | |
| Amoxicillin-clavulanate | R | | | | | |
| Cefazolin | R | | | | | |
| Cefuroxime | R | | | | | |
| Cefotaxime | R | | | | | |
| Ceftriaxone | R | | | | | |
| Ceftazidime | R | | | | | |
| Cefepime | R | | | | | |
| Aztreonam | R | | | | | |
| Cefoxitin | R | | | | | |
| Cefotetan | R | | | | | |
| Meropenem | R | | | | | |
| Ertapenem | R | | | | | |
| Imipenem | R | | | | | |
| Gentamicin | | R | R | R | | |
| Tobramycin | | R | R | S | | |
| Amikacin | | R | S | S | | |
| Ciprofloxacin | R | | | | | |
| Levofloxacin | R | | | | | |
| Tetracycline | | | | | | R |
| TMP-SMZ | | | | | R | |

When the test sample is located out of the ST11 cluster, its resistance profiles to 23 antibacterial drugs including ampicillin-sulbactam, piperacillin-tazobactam, cefoperazone-sulbactam, amoxicillin-clavulanate, cefazolin, cefuroxime, cefotaxime, ceftriaxone, ceftazidime, cefepime, aztreonam, cefoxitin, cefotetan, meropenem, ertapenem, imipenem, gentamicin, tobramycin, amikacin, ciprofloxacin, levofloxacin, tetracycline and TMP-SMZ is inferred according to the following ST11-type interpretation rules, that is, the drug resistance profile predicted by phylogenetic tree interpretation is preferred over antimicrobial resistance determinants analysis, and the test sample is directly determined to be resistant to cephalosporins, cephamycins, carbapenems and quinolones. The drug species and antimicrobial resistance determinants used in this study are shown in Table 10.

**Table 11**

| Antibiotics | ST23 phyloge netic tree inferrence | ST15 phyloge netic tree inferrence | Antimicrobial resistance determinants | | | | | | | | | | |
|---|---|---|---|---|---|---|---|---|---|---|---|---|---|
| | | | *CT X & TE M* | *C T X* | *D HA* | *KP C* | *rm tB* | *aac( 6')-I b3* | *aph( 3')-I α* | *Qn rS* | *Q R D R* | *sul1 sul2 sul3* | *tet A* |
| Ampicilli n-sulbact am | S | R | R | R | | R | | | | | | | |
| Piperacill in-tazoba ctam | S | R | | | | R | | | | | | | |
| Cefopera zone-sulb actam | S | R | | | | R | | | | | | | |
| Amoxicil lin-clavul anate | S | R | R | R | | R | | | | | | | |
| Cefazolin | S | R | R | R | | R | | | | | | | |
| Cefuroxi me | S | R | R | R | | R | | | | | | | |
| Cefotaxi me | S | R | R | R | | R | | | | | | | |
| Ceftriaxo ne | S | R | R | R | | R | | | | | | | |
| Ceftazidi me | S | R | R | | | R | | | | | | | |
| Cefepime | S | R | R | | | R | | | | | | | |
| Aztreona m | S | R | R | R | | R | | | | | | | |
| Cefoxitin | S | R | | | R | R | | | | | | | |
| Cefotetan | S | R | | | R | R | | | | | | | |
| Meropen em | S | R | | | | R | | | | | | | |
| Ertapene m | S | R | | | | R | | | | | | | |
| Imipene m | S | R | | | | R | | | | | | | |
| Gentamic in | | | | | | | R | R | R | | | | |
| Tobramy cin | | | | | | | R | R | S | | | | |
| Amikaci n | | | | | | | R | S | S | | | | |
| Ciproflox acin | | R | | | | | | | | R | R | | |
| Levoflox acin | | R | | | | | | | | R | R | | |
| Tetracycline | | | | | | | | | | | | | R |
| TMP-SM Z | | | | | | | | | | | | R | |

### 3. Database validation and result interpretation

(1) Blind test: The genome-wide SNPs loci of library *Klebsiella pneumoniae* and 24 blinds were identified using kSNP3 (Version 3.1) (Standard mode, kmer=31), and a genomic phylogenetic tree based on SNP similairties were generated for cluster analysis. As is shown in FIGS. 12A to 12C, there were 10 blinds attributed to the ST11 cluster, and 14 blinds belonged to the non-STll clusters, including 1 case of ST15 type, 2 cases of ST23 type, and 11 cases of other ST types. The resistance profiles of each blind were determined according to the prediction rules of ST11, ST15, ST23, and non-ST11-ST15-ST23 types, respectively. 7 blinds were identified as susceptible to all antibiotics involved in this study, and none of them contained any antimicrobial resistance determinants; the rest were pandrug-resistant or multidrug resistant.
(2) Result interpretation: Based on the positioning in the phylogenetic tree, the antimicrobial resistance determinants and the corresponding prediction rules, the resistance profiles of 24 blinds against 23 antibiotics were determined and compared with the gold standard VITEK2 AST result, as shown in Table 12.

**Table 12**

| Antibiotics | Resistanc e profiles by VITEK2 | | Resistanc e profiles by the present method | | Positive predictiv e value (PPV) | Negative predictiv e value (NPV) | Sentitivit y | Specificit y |
|---|---|---|---|---|---|---|---|---|
| | R | S | R | S | | | | |
| Ampicillin-sulbactam | 15 | 9 | 15 | 9 | 100.00% | 100.00% | 100.00% | 100.00% |
| Piperacillin-tazobactam | 13 | 11 | 12 | 12 | 100.00% | 91.67% | 92.31% | 100.00% |
| Cefoperazone-sulbacta m | 13 | 11 | 12 | 12 | 100.00% | 91.67% | 92.31% | 100.00% |
| Amoxicillin-clavulanate | 16 | 8 | 16 | 8 | 100.00% | 100.00% | 100.00% | 100.00% |
| Cefazolin | 15 | 9 | 15 | 9 | 100.00% | 100.00% | 100.00% | 100.00% |
| Cefuroxime | 15 | 9 | 15 | 9 | 100.00% | 100.00% | 100.00% | 100.00% |
| Cefotaxime | 15 | 9 | 15 | 9 | 100.00% | 100.00% | 100.00% | 100.00% |
| Ceftriaxone | 15 | 9 | 15 | 9 | 100.00% | 100.00% | 100.00% | 100.00% |
| Ceftazidime | 13 | 11 | 12 | 12 | 100.00% | 91.67% | 92.31% | 100.00% |
| Cefepime | 13 | 11 | 12 | 12 | 100.00% | 91.67% | 92.31% | 100.00% |
| Aztreonam | 15 | 9 | 15 | 9 | 100.00% | 100.00% | 100.00% | 100.00% |
| Cefoxitin | 12 | 12 | 12 | 12 | 100.00% | 100.00% | 100.00% | 100.00% |
| Cefotetan | 12 | 12 | 12 | 12 | 100.00% | 100.00% | 100.00% | 100.00% |
| Meropenem | 12 | 12 | 12 | 12 | 100.00% | 100.00% | 100.00% | 100.00% |
| Ertapenem | 12 | 12 | 12 | 12 | 100.00% | 100.00% | 100.00% | 100.00% |
| Imipenem | 12 | 12 | 12 | 12 | 100.00% | 100.00% | 100.00% | 100.00% |
| Gentamicin | 12 | 12 | 12 | 12 | 100.00% | 100.00% | 100.00% | 100.00% |
| Tobramycin | 9 | 15 | 9 | 15 | 100.00% | 100.00% | 100.00% | 100.00% |
| Amikacin | 9 | 15 | 9 | 15 | 100.00% | 100.00% | 100.00% | 100.00% |
| Ciprofloxacin | 14 | 10 | 15 | 9 | 93.33% | 100.00% | 100.00% | 90.00% |
| Levofloxacin | 14 | 10 | 15 | 9 | 93.33% | 100.00% | 100.00% | 90.00% |
| Tetracycline | 11 | 13 | 11 | 13 | 100.00% | 100.00% | 100.00% | 100.00% |
| TMP-SMZ | 11 | 13 | 10 | 14 | 100.00% | 92.86% | 90.91% | 100.00% |

Among the 24 blinds, 1 case of false negative was found against piperacillin/tazobactam, cefoperazone/sulbactam, ceftazidime, cefpiramide, trimethoprim/sulfamethoxazole, respectively, and 1 case of false positive was found against ciprofloxacin and levofloxacin, respectively. In total, 5 false negative results and 2 false positive results were found, counting up 23 antibiotics (552 susceptibility results). When compared with the gold standard VITEK2 AST, the positive predictive value, negative predictive value, sensitivity and specificity of the present method was demonstrated to be 99.32% (293/295), 98.05% (252/257), 98.32% (293/298), and 99.21% (252/254), respectively. The performances meet the design requirements of the present method and the needs of clinical application, that is, the sensitivity and specificity being above 95%.

### Example 8: Construction and validation of a genomic phylogenetic tree database for Staphylococcus aureus based on WGS

*Staphylococcus aureus* was selected as the representative of Gram-positive cocci to illustrate the way a genomic phylogenetic tree for drug susceptibility determination is constructed. Other gram-positive cocci such as Coagulase-Negative *Staphylococcus, Enterococcus faecalis* can refer to this method for library construction and analysis.

### 1. Method

Step 1. Sample collection and drug susceptibility verification: 160 clinical *Staphylococcus aureus* isolates were collected from 20 hospitals across china in a period between June 2018 and June 2019. All isolates were tested for drug susceptibility using bioMérieux Vitek2 Compact System and AST-P639 card, and specifically, and the results were used as the gold standard (culture-based AST).

Step 2. Genomic DNA preparation: Strains were inoculated on Columbia sheep blood agar plates by streaking and placed in an incubator (37°C) for 24 hours. The bacterial precipitate was then collected by centrifugation at 10,000 rpm for 2 mins, and incubated with lysozyme at a final concentration of 20mg/mL at 37°C for 30-60min. The genomic DNA was purified from the pellets using DNeasy Blood and Tissue kit following the protocol by the manufacturer. DNA concentration was measured using Qubit fluorometer and the QC was performed on a NanoDropTM spectrophotometer, DNA with OD 260/280 1.6-2.0 and 260/230 2.0-2.2 were accepted for nanopore sequencing library preparation.

Step 3. Library preparation: ONT Native Barcoding Kit ID (EXP-NBD104 & 114) and Ligation Sequencing Kit ID (SQK LSK109) were used in library preparation following the ID Native barcoding genomic DNA protocol developed by ONT with a few modifications. The procedure is briefly described below, DNA extracted from each isolate were quantified on a Qubit 3.0 fluorometer and diluted to 20ng/µ1 with nuclease-free water (NF water), 50µl diluted DNA was used as starting material and incubated with 7µl Ultra II End-Prep reaction buffer and 3µl Ultra II End-Prep enzyme mix (New England Biolabs, USA) for 5 minutes at 20°C follow by 5 minutes at 65°C. The end-prepped DNA was then purified from the reaction mix using 1× (v/v) AMPure XP magnetic beads and eluted with 25µl NF water. After quantification, elution containing 500ng DNA was obtained from each sample and topped up to 22.5µl with NF water and mixed with 2.5µl unique Native Barcode and 25µl Blunt/TA Ligation Master Mix. The mixture was incubated at room temperature for 10min and purified with 1× (v/v) AMPure XP magnetic beads, and the barcoded DNA was eluted with 26µl NF water. In the final step, equal amounts of 12 individually barcoded DNA samples were pooled to 700ng in total, and extra NF water were added to 50µl final volume. To the pooled DNA, 20µl ONT Barcode Adapter Mix, 20µl NEBNext Quick Ligation Reaction Buffer (5X) and 10µl Quick T4 DNA Ligase was added in order and mixed thoroughly, and after 10 minutes incubation at room temperature, the BAM ligated DNA was purified from the reaction mix with 0.4× (v/v) AMPure XP magnetic beads, and eluted in 15µl NF water.

Step 4. Nanopore sequencing: The sequencing consumable used in this study was ONT flowcell FLO-MIN106 R9.4. After flowcell priming, 75µL prepared library (35µL Running buffer, 255µL loading beads, and 145µL pooled library) was loaded. Sequencing was performed on an ONT MinIONTM portable sequencing device, and set and monitored using ONT MinKNOWTM desk software. Samples were pooled and sequenced for about 6 hours or until 1Gb of data for each sample were generated for database isolates and clinical specimens, respectively.

Step 5. Bioinformatics analysis: Raw FAST5 reads files were base-called using the Guppy v3.2.4 basecalling software. And the CANU 2.0 software was used to assemble the reads in fastq files into genomic assemblies or contigs with default parameters. The assembled genomes were analyzed using the local antibiotic resistance database for resistance determinants identification. Called fastq files of clinical specimens were analyzed with the EPI2ME WIMP rev.3.3.1 pipeline for the identification of pathogens present in the sample and reads classified as *Staphylococcus aureus* were extracted for further resistance analysis. A local nBLAST tool was created for interrogating contigs of clinical samples for a panel of validated AMR determinants. Phylogenetic analysis was conducted with kSNP3 (version 3.1) based on pan-genome SNPs identified and a Kmer value of 31 was adopted.

### 2. Database construction and analytical logic design

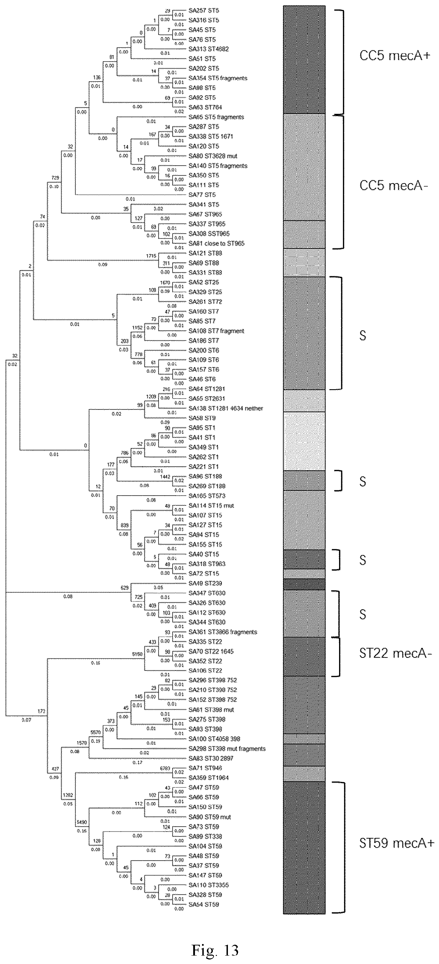

(1) Construction of a genomic phylogenetic tree: Through biomarker analysis, identical clones were merged and 93 representative *Staphylococcus aureus* clones were selected for the database and a genomic phylogenetic tree database was built as shown in FIG. 13. The branches of the phylogenetic tree representing different resistance profiles are indicated by different colors on the right.
(2) Aalytical logic design
   (2a) Species identification: When the genome assembly size of the test sample is within the range of 2,400,000-3,000,000bp, fragments of different lengths from multiple sites in the genome were selected to BLAST against the NCBI nucleotide database. The species of the pathogen is determined as *Staphylococcus aureus* only when the strain description shows *Staphylococcus aureus* and the per identity value exceeds 98%.
   (2b) Drug susceptibility determination: When the test sample is located in the CC5mecA+ or ST59mecA+ clusters, follow the principle that the drug resistance profile predicted by phylogenetic tree interpretation is preferred over antimicrobial resistance determinants analysis, and the sample is directly determined to be resistant to penicillin, oxacillin, cefoxitin and quinolones. When the test sample is located in the CC5mecA- or ST22mecA- clusters, follow the principle that the drug resistance profile predicted by phylogenetic tree interpretation is preferred over antimicrobial resistance determinants analysis, and the sample is directly determined to be susceptible to penicillin, oxacillin, and cefoxitin. When the test sample is located in the susceptibility branches of the genomic phylogenetic tree, follow the principle that the drug resistance profile predicted by phylogenetic tree interpretation is preferred over antimicrobial resistance determinants analysis, and the sample is directly determined to be susceptible to all antibiotics involved in this study. When the test sample is located in branches outside the above designated regions, the drug resistance profile is determined solely by antimicrobial resistance determinants.

The drug species and antimicrobial resistance determinants used in this study are shown in Table 13.

**Table 13**

| | | Pe nici llin | Ox acil lin, Cef oxi tin | Qui nolo nes | Ma crol ides | Clin dam ycin | I C R | TM P-S MZ | Lin ezo lid | Van com ycin | Teic opla nin | Tetr acyc lines | Rifa mpi cin | Gen tami cin |
|---|---|---|---|---|---|---|---|---|---|---|---|---|---|---|
| Resu lts by phyl ogen etic tree | CC5m ecA+ | R | R | R | | | | | | | | | | |
| | CC5m ecA- | S | S | | | | | | | | | | | |
| | ST59 mecA + | R | R | R | | | | | | | | | | |
| | ST22 mecA- | S | S | | | | | | | | | | | |
| | S | S | S | S | S | S | S | S | S | S | S | S | S | S |
| Resu Its by anti micr obial resis tanc e deter mina nts | *blaZ* | R | | | | | | | | | | | | |
| | *mecA* | R | R | | | | | | | | | | | |
| | *gyrA*/*p arC* | | | R | | | | | | | | | | |
| | *ermAe rmB* | | | | R | R | R | | | | | | | |
| | *ermC* | | | | R | | R | | | | | | | |
| | *mphC*/ *msrA* | | | | R | | | | | | | | | |
| | *dfrG* | | | | | | | R | | | | | | |
| | *23s rRNA* | | | | | | | | R | | | | | |
| | *vanA* | | | | | | | | | R | R | | | |
| | *tetL*/*tet M*/*tetK* | | | | | | | | | | | R | | |
| | *rpoB* | | | | | | | | | | | | R | |
| | *AAC6-Ie-AP H2-Ia* | | | | | | | | | | | | | R |

### 3. Database validation and result interpretation

(1) Blind test: The genome-wide SNPs loci of library *Staphylococcus aureus* and 22 blinds were identified using kSNP3 (Version 3.1) (Standard mode, kmer=31), and a genomic phylogenetic tree based on SNP similairties were generated for cluster analysis. As is shown in FIG. 13, there were 3 blinds attributed to the CC5mecA+ cluster, 2 blinds belonged to the CC5mecA- cluster, 4 blinds belonged to the ST59mecA+ cluster, and 5 blinds belonged to the S cluster. The resistance profiles of each blind were determined according to the prediction rules of CC5mecA+, CC5mecA-, ST59mecA+, and S types, respectively, following the principle that the drug resistance profile predicted by phylogenetic tree interpretation is preferred over antimicrobial resistance determinants analysis.
(2) Result interpretation: Based on the positioning in the phylogenetic tree, the antimicrobial resistance determinants and the corresponding prediction rules, the resistance profiles of 22 blinds against 20 antibiotics were determined and compared with the gold standard VITEK2 AST result, as shown in Table 14 (For antibiotics without relaveant drug-resistant samples, the positive predictive value and sensitivity are not counted).

**Table 14**

| Antibiotics | Resistance profiles by VITEK2 | | Resistance profiles by the present method | | Positive predictive value (PPV) | Negative predictive value (NPV) | Sentitivity | Specificity |
|---|---|---|---|---|---|---|---|---|
| | R | S | R | S | | | | |
| Penicillin | 20 | 2 | 20 | 2 | 100.00% | 100.00% | 100.00% | 100.00% |
| Oxacillin, Cefoxitin | 8 | 14 | 8 | 14 | 100.00% | 100.00% | 100.00% | 100.00% |
| Quinolones | 6 | 16 | 6 | 16 | 100.00% | 100.00% | 100.00% | 100.00% |
| Macrolides | 15 | 7 | 14 | 8 | 100.00% | 87.50% | 93.33% | 100.00% |
| Clindamycin | 10 | 12 | 9 | 13 | 100.00% | 92.31% | 90.00% | 100.00% |
| ICR | 15 | 7 | 14 | 8 | 100.00% | 87.50% | 93.33% | 100.00% |
| Cefazolin | 0 | 22 | 0 | 22 | - | 100.00% | - | 100.00% |
| Vancomycin | 0 | 22 | 0 | 22 | - | 100.00% | - | 100.00% |
| Teicoplanin | 0 | 22 | 0 | 22 | - | 100.00% | - | 100.00% |
| Linezolid | 0 | 22 | 0 | 22 | - | 100.00% | - | 100.00% |
| TMP-SMZ | 5 | 17 | 5 | 17 | 100.00% | 100.00% | 100.00% | 100.00% |
| Tetracycline | 6 | 16 | 6 | 16 | 100.00% | 100.00% | 100.00% | 100.00% |
| Tigecycline | 0 | 22 | 0 | 22 | - | 100.00% | - | 100.00% |
| Rifampicin | 1 | 21 | 1 | 21 | 100.00% | 100.00% | 100.00% | 100.00% |
| Gentamicin | 7 | 15 | 7 | 15 | 100.00% | 100.00% | 100.00% | 100.00% |

Among the 22 blinds, 1 case of false negative was found against macrolides (erythromycin, clarithromycin, azithromycin), and lincosamides (clindamycin, ICR); no false positive case was found. In total, 5 false negative results and were found, counting up 20 antibiotics (440 susceptibility results). When compared with the gold standard VITEK2 AST, the positive predictive value, negative predictive value, sensitivity and specificity of the present method was demonstrated to be 100.00% (138/138), 98.34% (297/302), 96.50% (138/143), and 100.00% (297/297), respectively. The performances meet the design requirements of the present method and the needs of clinical application, that is, the sensitivity and specificity being above 95%.

### Example 9: Construction and validation of a genomic phylogenetic tree database for Streptococcus pneumoniae based on WGS

*Streptococcus pneumoniae* was selected as the representative of the fastidious bacteria to illustrate the way a genomic phylogenetic tree for drug susceptibility determination is constructed.

### 1. Method

Step 1. Sample collection and drug susceptibility verification: 48 clinical *Streptococcus pneumoniae* isolates were collected from 18 hospitals across china in a period between May 2017 and October 2019. All isolates were tested for drug susceptibility using bioMérieux Vitek2 Compact System and AST-GP68 card, and specifically, the susceptibility of penicillin was double checked by disc diffusion method (OXOID, CT0043B), and the combined results were used as the gold standard (culture-based AST).

Step 2. Genomic DNA preparation: Strains were inoculated on Columbia sheep blood agar plates by streaking and placed in an incubator (37°C) for 24 hours. The bacterial precipitate was then collected by centrifugation at 10,000 rpm for 2 mins, and incubated with lysozyme at a final concentration of 20mg/mL at 37°C for 30-60min. The genomic DNA was purified from the pellets using DNeasy Blood and Tissue kit following the protocol by the manufacturer. DNA concentration was measured using Qubit fluorometer and the QC was performed on a NanoDropTM spectrophotometer, DNA with OD 260/280 1.6-2.0 and 260/230 2.0-2.2 were accepted for nanopore sequencing library preparation.

Step 3. Library preparation: ONT Native Barcoding Kit ID (EXP-NBD104 & 114) and Ligation Sequencing Kit ID (SQK LSK109) were used in library preparation following the ID Native barcoding genomic DNA protocol developed by ONT with a few modifications. The procedure is briefly described below, DNA extracted from each isolate were quantified on a Qubit 3.0 fluorometer and diluted to 20ng/µ1 with nuclease-free water (NF water), 50µl diluted DNA was used as starting material and incubated with 7µl Ultra II End-Prep reaction buffer and 3µl Ultra II End-Prep enzyme mix (New England Biolabs, USA) for 5 minutes at 20°C follow by 5 minutes at 65°C. The end-prepped DNA was then purified from the reaction mix using 1× (v/v) AMPure XP magnetic beads and eluted with 25µl NF water. After quantification, elution containing 500ng DNA was obtained from each sample and topped up to 22.5µl with NF water and mixed with 2.5µl unique Native Barcode and 25µl Blunt/TA Ligation Master Mix. The mixture was incubated at room temperature for 10min and purified with 1× (v/v) AMPure XP magnetic beads, and the barcoded DNA was eluted with 26µl NF water. In the final step, equal amounts of 12 individually barcoded DNA samples were pooled to 700ng in total, and extra NF water were added to 50µl final volume. To the pooled DNA, 20µl ONT Barcode Adapter Mix, 20µl NEBNext Quick Ligation Reaction Buffer (5X) and 10µl Quick T4 DNA Ligase was added in order and mixed thoroughly, and after 10 minutes incubation at room temperature, the BAM ligated DNA was purified from the reaction mix with 0.4× (v/v) AMPure XP magnetic beads, and eluted in 15µl NF water.

Step 4. Nanopore sequencing: The sequencing consumable used in this study was ONT flowcell FLO-MIN106 R9.4. After flowcell priming, 75µL prepared library (35µL Running buffer, 25.5µL loading beads, and 14.5µL pooled library) was loaded. Sequencing was performed on an ONT MinIONTM portable sequencing device, and set and monitored using ONT MinKNOWTM desk software. Samples were pooled and sequenced for about 6 hours or until 1Gb of data for each sample were generated for database isolates and clinical specimens, respectively.

Step 5. Bioinformatics analysis: Raw FAST5 reads files were base-called using the Guppy v3.2.4 basecalling software. And the CANU 2.0 software was used to assemble the reads in fastq files into genomic assemblies or contigs with default parameters. The assembled genomes were analyzed using the local antibiotic resistance database for resistance determinants identification. Called fastq files of clinical specimens were analyzed with the EPI2ME WIMP rev.3.3.1 pipeline for the identification of pathogens present in the sample and reads classified as *Streptococcus pneumoniae* were extracted for further resistance analysis. A local nBLAST tool was created for interrogating contigs of clinical samples for a panel of validated AMR determinants. Phylogenetic analysis was conducted with kSNP3 (version 3.1) based on pan-genome SNPs identified and a Kmer value of 31 was adopted.

### 2. Database construction and analytical logic design

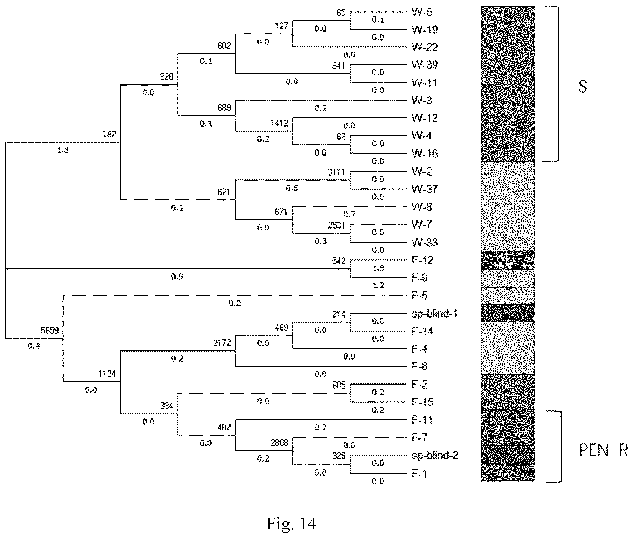

(1) Construction of a genomic phylogenetic tree: Through biomarker analysis, identical clones were merged and 25 representative *Streptococcus pneumoniae* clones were selected for the database and a genomic phylogenetic tree database was built as shown in FIG. 14. The branches of the phylogenetic tree representing different resistance profiles are indicated by different colors on the right, where yellow indicates susceptible to all antibiotics, blue indicates penicillin-resistant, and black represents blind samples.
(2) Aalytical logic design
   (2a) Species identification: When the genome assembly size of the test sample is within the range of 2,000,000-2,300,000bp, fragments of different lengths from multiple sites in the genome were selected to BLAST against the NCBI nucleotide database. The species of the pathogen is determined as *Streptococcus pneumoniae* only when the strain description shows *Streptococcus pneumoniae* and the per identity value exceeds 98%.
   (2b) Drug susceptibility determination: When the test sample is located in the PEN-R cluster, follow the principle that the drug resistance profile predicted by phylogenetic tree interpretation is preferred over antimicrobial resistance determinants analysis, and the sample is directly determined to be resistant to penicillin and cephalosporins. When the test sample is located in the S branch of the genomic phylogenetic tree, follow the principle that the drug resistance profile predicted by phylogenetic tree interpretation is preferred over antimicrobial resistance determinants analysis, and the sample is directly determined to be susceptible to all antibiotics involved in this study. When the test sample is located in branches outside the above designated regions, the drug resistance profile is determined solely by antimicrobial resistance determinants.

The drug species and antimicrobial resistance determinants used in this study are shown in Table 15.

**Table 15**

| | | Pe nic illi n | Am oxi cilli n | Ce fep im e | Cef ota xim e | Cef tria xon e | Ert ape ne m | Me rop ene m | Eryt hro myc in | TM P-S MZ | Lev oflo xaci n | Mo xifl oxa cin | Van com ycin | Li ne zol id | Tetr acyc line s |
|---|---|---|---|---|---|---|---|---|---|---|---|---|---|---|---|
| Res ults by phyl oge neti c tree | PE N-R | R | R | R | R | R | | | | | | | | | |
| | S | S | S | S | S | S | S | S | S | S | S | S | S | S | S |
| Res ults by anti micr obia 1 resis tanc e dete rmi nant | *pb p2x* | R | R | R | R | R | | | | | | | | | |
| | *pb p1 a* | R | R | R | R | R | | | | | | | | | |
| | *pb p2 b* | R | R | R | R | R | | | | | | | | | |
| | *er mA* /*B*/ *C* | | | | | | | | R | | | | | | |
| | *Isa* | | | | | | | | R | | | | | | |
| s | *A*/ *E* | | | | | | | | | | | | | | |
| | *dfr* | | | | | | | | | R | | | | | |
| | *tet L*/*t et M* | | | | | | | | | | | | | | R |
| | *23s rR NA* | | | | | | | | | | | | | R | |
| | *gyr A*/*p ar C* | | | | | | | | | | R | R | | | |

### 3. Database validation and result interpretation

(1) Blind test: The genome-wide SNPs loci of library *Streptococcus pneumoniae* and 2 blinds were identified using kSNP3 (Version 3.1) (Standard mode, kmer=31), and a genomic phylogenetic tree based on SNP similairties were generated for cluster analysis. As is shown in FIG. 14, the blind 1 was belonged to neither PEN-R nor S cluster, and its drug resistance profile is determined solely by antimicrobial resistance determinants; the blind 2 was located in the PEN-R cluster, and its drug resistance profile is directly determined to be resistant to penicillin and cephalosporins, following the principle that the drug resistance profile predicted by phylogenetic tree interpretation is preferred over antimicrobial resistance determinants analysis.
(2) Result interpretation: Based on the positioning in the phylogenetic tree, the antimicrobial resistance determinants and the corresponding prediction rules, the resistance profiles of 2 blinds against 14 antibiotics were determined and compared with the gold standard VITEK2 AST result, as shown in Table 16.

**Table 16**

| Sample | penici llin | cefepime, cefotaxime , ceftriaxone | ert ap en em | me rop ene m | eryt hro myci n | TM P-S MZ | vanc omy cin | linez olid | tetrac ycline | levoflo xacin, moxiflo xacin |
|---|---|---|---|---|---|---|---|---|---|---|
| Blind 1 by this method | | | | | erm B | dfr | | | tetM | |
| | S | S | S | S | R | R | S | S | R | S |
| Blind 1 by AST | S | S | S | S | R | R | S | S | R | S |
| blind 2 by this method | PEN-R | PEN-R | | | erm B | dfr | | | tetM | |
| | R | R | S | S | R | R | S | S | R | S |
| blind 2 by AST | R | R | S | S | R | R | S | S | R | S |

By analyzing the sample positioning in the phylogenetic tree, combined with antimicrobial resistance determinants and specific prediction rules, the susceptibility of the two blinds inferred by this present method showed a 100% agreement with the gold standard VITEK2 AST result.

### Example 10: Construction and validation of a genomic phylogenetic tree database for Candida albicans based on WGS

*Candida albicans* was selected as the representative of fungi to illustrate the way a genomic phylogenetic tree for drug susceptibility determination is constructed. Other *Candida spp.* or yeast-like fungi can refer to this method for library construction and analysis.

### 1. Method

Step 1. Sample collection and drug susceptibility verification: 120 clinical *Candida albicans* isolates were collected from 31 hospitals across china in a period between September 2015 and January 2019. All isolates were tested for drug susceptibility using broth microdilution method following the M27-A3 and M27-S4 guidelines of NCCLS, and the results were used as the the gold standard (culture-based AST).

Step 2. Genomic DNA preparation: Strains were inoculated on Sabouraud plate or Chromogenic agar plate by streaking and placed in an incubator (37°C) for 24 hours. The fungal precipitate was then collected by centrifugation at 10,000 rpm for 2 mins, and incubated with sorbitol sodium phosphate buffer and lysozyme at a final concentration of 1.2 mol/L and 20mg/mL, respectively, at 37°C for 30-60min. The genomic DNA was purified from the pellets using DNeasy Blood and Tissue kit following the protocol by the manufacturer. DNA concentration was measured using Qubit fluorometer and the QC was performed on a NanoDropTM spectrophotometer, DNA with OD 260/280 1.6-2.0 and 260/230 2.0-2.2 were accepted for nanopore sequencing library preparation.

Step 3. Library preparation: ONT Native Barcoding Kit ID (EXP-NBD104 & 114) and Ligation Sequencing Kit ID (SQK LSK109) were used in library preparation following the ID Native barcoding genomic DNA protocol developed by ONT with a few modifications. The procedure is briefly described below, DNA extracted from each isolate were quantified on a Qubit 3.0 fluorometer and diluted to 20ng/µl with nuclease-free water (NF water), 50µl diluted DNA was used as starting material and incubated with 7µl Ultra II End-Prep reaction buffer and 3µl Ultra II End-Prep enzyme mix (New England Biolabs, USA) for 5 minutes at 20°C follow by 5 minutes at 65°C. The end-prepped DNA was then purified from the reaction mix using 1× (v/v) AMPure XP magnetic beads and eluted with 25µl NF water. After quantification, elution containing 500ng DNA was obtained from each sample and topped up to 22.5µl with NF water and mixed with 2.5µl unique Native Barcode and 25µl Blunt/TA Ligation Master Mix. The mixture was incubated at room temperature for 10min and purified with 1× (v/v) AMPure XP magnetic beads, and the barcoded DNA was eluted with 26µl NF water. In the final step, equal amounts of 12 individually barcoded DNA samples were pooled to 700ng in total, and extra NF water were added to 50µl final volume. To the pooled DNA, 20µl ONT Barcode Adapter Mix, 20µl NEBNext Quick Ligation Reaction Buffer (5X) and 10µl Quick T4 DNA Ligase was added in order and mixed thoroughly, and after 10 mins incubation at room temperature, the BAM ligated DNA was purified from the reaction mix with 0.4× (v/v) AMPure XP magnetic beads, and eluted in 15µl NF water.

Step 4. Nanopore sequencing: The sequencing consumable used in this study was ONT flowcell FLO-MIN106 R9.4. After flowcell priming, 75µE prepared library (35µL Running buffer, 25.5µL loading beads, and 14.5µL pooled library) was loaded. Sequencing was performed on an ONT MinIONTM portable sequencing device, and set and monitored using ONT MinKNOWTM desk software. Samples were pooled and sequenced for about 6 hours or until 1Gb of data for each sample were generated for database isolates and clinical specimens, respectively.

Step 5. Bioinformatics analysis: Raw FAST5 reads files were base-called using the Guppy v3.2.4 basecalling software. And the CANU 2.0 software was used to assemble the reads in fastq files into genomic assemblies or contigs with default parameters. The assembled genomes were analyzed using the local antibiotic resistance database for resistance determinants identification. Called fastq files of clinical specimens were analyzed with the EPI2ME WIMP rev.3.3.1 pipeline for the identification of pathogens present in the sample and reads classified as *Candida albicans* were extracted for further resistance analysis. A local nBLAST tool was created for interrogating contigs of clinical samples for a panel of validated AMR determinants. Phylogenetic analysis was conducted with kSNP3 (version 3.1) based on pan-genome SNPs identified and a Kmer value of 31 was adopted.

### 2. Database construction and analytical logic design

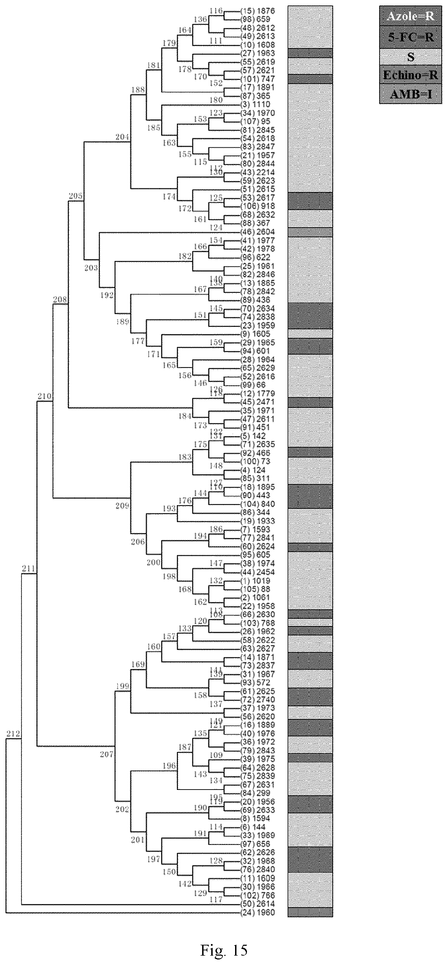

(1) Construction of a genomic phylogenetic tree: Through biomarker analysis, identical clones were merged and 107 representative *Candida albicans* clones were selected for the database and a genomic phylogenetic tree database was built as shown in FIG. 15. The branches of the phylogenetic tree representing different resistance profiles are indicated by different colors on the right, where red indicates azole-resistant, yellow indicates 5-fluorocytosine-resistant, green indicates echinocandins-resistant, gray indicates Amphotericin-B-intermediate, and blue indicates susceptible to all antibiotics.
(2) Aalytical logic design
(2a) Species identification: When the genome assembly size of the test sample is within the range of 12,000,000-16,000,000bp, fragments of different lengths from multiple sites in the genome were selected to BLAST against the NCBI nucleotide database. The species of the pathogen is determined as *Candida albicans* only when the strain description shows *Candida albicans* and the per identity value exceeds 98%.
(2b) Drug susceptibility determination: Resistance of *Candida albicans* to triazoles and amphotericin B formulations is determined by the positioning of a test sample in the genomic phylogenetic tree, following the principle that the drug resistance profile predicted by phylogenetic tree interpretation is preferred over antimicrobial resistance determinants analysis; resistance of *Candida albicans* to echinocandins is determined by antimicrobial resistance determinants analysis. The drug species and antimicrobial resistance determinants used in this study are shown in Table 17.

**Table 17**

| Antibiotics | Results by phylogenetic tree | Antimicrobial resistance determinants | | | | | |
|---|---|---|---|---|---|---|---|
| | | *fcy2* | *fca1* | *fur1* | *erg11* | *fks1* | *fks2* |
| Amphotericin B | S | | | | | | |
| 5-Fluorocytosine | | R | R | R | | | |
| Fluconazole | | | | | R | | |
| Itraconazole | | | | | R | | |
| Voriconazole | | | | | R | | |
| Caspofungin | | | | | | R | R |
| Micafungin | | | | | | R | R |

### 3. Database validation and result interpretation

(1) Blind test: The genome-wide SNPs loci of library *Candida albicans* and 12 blinds were identified using kSNP3 (Version 3.1) (Standard mode, kmer=31), and a genomic phylogenetic tree based on SNP similairties were generated for cluster analysis. As is shown in FIG. 15, none of the 12 blinds was belonged to Amphotericin B-R or echinocandin-R cluster. The phylogenetic tree prediction and Antimicrobial resistance determinants analysis of the 12 blinds are detailed in Table 18:

**Table 18**

| | Results by phylogenetic tree Amphotericin B | Antimicrobial resistance determinants (Amino acid substitution) | | | | | | |
|---|---|---|---|---|---|---|---|---|
| | | Echinoc andin | *fcy2* | *fca1* | *fur1* | *erg11* | *Jks1* | *fks2* |
| lind-1 | S | S | - | - | - | - | - | - |
| lind-2 | S | S | H80R | - | - | - | - | - |
| lind-3 | S | S | - | - | - | - | - | - |
| lind-4 | S | S | - | G28D | - | - | - | - |
| lind-5 | S | S | - | - | - | Y132H+R467 K | - | - |
| lind-6 | S | S | - | G28D heterogene ous | - | - | - | - |
| lind-7 | S | S | - | - | - | - | - | - |
| lind-8 | S | S | - | - | - | - | - | - |
| lind-9 | S | S | - | - | - | K259E+I471T | - | - |
| lind-10 | S | S | - | - | - | - | - | - |
| lind-11 | S | S | - | - | R101 C | - | - | - |
| lind-12 | S | S | - | - | - | - | - | - |

(2) Result interpretation: Based on the positioning in the phylogenetic tree, the antimicrobial resistance determinants and the corresponding prediction rules, the resistance profiles of 12 blinds against 7 antibiotics were determined and compared with the the gold standard broth microdilution method (culture-based AST), as shown in Table 19 (For antibiotics without relaveant drug-resistant samples, the positive predictive value and sensitivity are not counted).

**Table 19**

| Antibiotic | Results by broth microdilution method | | Results by this present method | | Positive predictive value (PPV) | Negative predictive value (NPV) | Sentitivity | Specificity |
|---|---|---|---|---|---|---|---|---|
| | R | S | R | S | | | | |
| 5-Fluorocytosine | 4 | 8 | 4 | 8 | 100.00% | 100.00% | 100.00% | 100.00% |
| Amphotericin B | 0 | 12 | 0 | 12 | - | 100.00% | - | 100.00% |
| Fluconazole | 2 | 10 | 2 | 10 | 100.00% | 100.00% | 100.00% | 100.00% |
| Itraconazole | 2 | 10 | 2 | 10 | 100.00% | 100.00% | 100.00% | 100.00% |
| Voriconazole | 2 | 10 | 2 | 10 | 100.00% | 100.00% | 100.00% | 100.00% |
| Caspofungin | 0 | 12 | 0 | 12 | - | 100.00% | - | 100.00% |
| Micafungin | 0 | 12 | 0 | 12 | - | 100.00% | - | 100.00% |

By analyzing the sample positioning in the phylogenetic tree, combined with antimicrobial resistance determinants and specific prediction rules, the resistance profiles of the 12 blinds inferred by this present method showed a 100% agreement with that of the gold standard broth microdilution method.

### Example 11: Rapid identification and antibiotic susceptibility inference with clinical metagenomics based on Nanopore sequencing technology and the rapid library method

A case of a clinical respiratory sputum specimen containing *Acinetobacter baumannii,* colletect in July 2019, was selected as the representative to illustrate the way a clinical sample is test and analyzed. Other non-fermenting Gram-negative bacteria can refer to this method for pathogen identification and drug susceptibility determination.

### 1. Method

(1) Take 1ml of sputum sample and add the same amount of the Digestion buffer, mix gently at room temperature for 15 mins. Recover 1ml of liquefied sputum into a 1.5ml tube, centrifuge at 12,000 rpm for 5 mins, discard the supernatant, and add 1ml of PBS buffer to fully resuspend the pellet. Transfer the supernatant to a clean 1.5ml tube, centrifuge at 12000×g for 5min, and discard the supernatant;
(2) Add 500 µl 1× PBS to resuspend the pellet, centrifuge at 12000×g for 5 min, and discard the supernatant;
(3) Add 98 µl of ddH2O and 2 µl of 5% saponin to the precipitate, mix by pipetting with a pipette tip, and let it stand for 10 mins at room temperature;
(4) Add 500 µl of 1× PBS, mix by pipetting with a pipette tip, and centrifuge at 12000×g for 5 min. Discard the supernatant;
(5) Add 40µl 1× PBS to resuspend the pellet, prepare the reaction solution according to the following reaction system, mix well and incubate at 37°C for 15min;

| Components | Volume |
|---|---|
| Sample | 40µl |
| 10 x reaction buffer | 5.6µl |
| Thermo DNase | 10µl |
| ddH2O | 0.5µl |

(6) Use the Bacterial Genomic DNA Extraction kit (Tiangen, China) to extract and purify the genomic DNA from the sample, and elute with 50ul TE;
(7) Take 2ul of the extracted genomic DNA for quantification with Qubit reagent.

### 2. Library preparation

(1) Prepare the End-prep reaction system according to the following table in a 0.2ml PCR reaction tube:

| Components | Volume |
|---|---|
| DNA | 45µl |
| Ultra II End-prep reaction buffer | 7µl |
| Ultra II End-prep enzyme mix | 3µl |
| ddH20 | 5µl |

(2) Mix gently by finger tapping, centrifuge briefly, incubate at 20°C for 5 mins, and then incubate at 65°C for 5 mins. Add AMPure XP (Beckman) magnetic beads that have been mixed and equilibrated to room temperature, and transfer the mixture to a 1.5ml tube. Invert and mix for 5min;
(3) After instant centrifugation, place the centrifuge tube on a magnetic rack, remove the supernatant after the magnetic beads are enriched, and wash twice with 200ul freshly-prepared 70% ethanol;
(4) Dry the magnetic beads at room temperature for 2 mins, and add 31ul TE buffer. After mixing and incubating for 2 mins, place the tube on the magnetic frame again. After the magnetic beads are aggregated, take the supernatant for use;
(5) Prepare the barcoding reaction solution according to the following table in a 1.5ml tube:

| Components | Volume |
|---|---|
| End-preped DNA | 30µl |
| Barcode Adapter | 20µl |
| Blunt/TA Ligase Master Mix | 50µl |

(6) Gently mix by finger tapping, centrifuge briefly and incubate at room temperature for 10 mins, add 100ul XP magnetic beads, mix by pipetting, and continue to invert and mix at room temperature for 5 minutes;
(7) After instant centrifugation, place the centrifuge tube on a magnetic rack, remove the supernatant after the magnetic beads are enriched, and wash twice with 200ul freshly prepared 70% ethanol;
(8) Dry the magnetic beads at room temperature for 2 min, and add 25ul TE buffer. Resuspend the beads and incubate at room temperature for 2 min, put the centrifuge tube back on the magnetic rack, and take the supernatant after the magnetic beads aggregate;
(9) Take 1ul of the eluate for quantification with Qubit reagent;
(10) Prepare the PCR reaction solution in a 0.2ml PCR tube as follows:

| Components | Volume | |
|---|---|---|
| PCR Barcode1 | 2µl | |
| 10 ng/µl adapter ligated template | | 2µl |
| LongAmpTaq 2x master mix | | 50µl |
| Nuclease-free water | | 46ul |

(11) PCR under the following conditions: 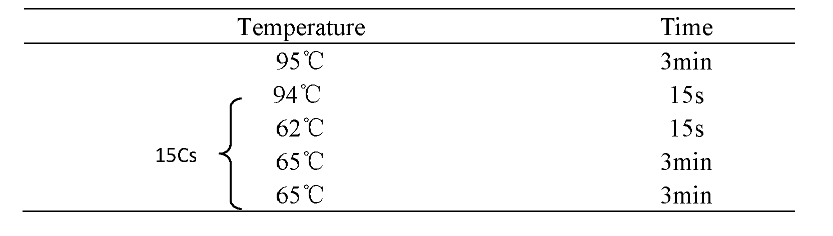
(12) Add 100ul XP magnetic beads, mix by pipetting, and invert and mix at room temperature for 5 minutes;
(13) After instant centrifugation, place the centrifuge tube on a magnetic stand, remove the supernatant once the solution turns clear, and wash twice with 200ul freshly prepared 70% ethanol;
(14) Dry the magnetic beads at room temperature for 2 minutes, add 46ul TE buffer. After mixing and incubating for 2 minutes off the rack, place the centrifuge tube on the magnetic rack again. Once the solution turns clear, take the supernatant for use;
(15) Take 1ul of the eluate for quantification with Qubit reagent;
(16) Prepare an End-prep reaction system in a 0.2ml PCR reaction tube according to the table below:

| Components | Volume |
|---|---|
| DNA | 45µl |
| Ultra II End-prep reaction buffer | 7µl |
| Ultra II End-prep enzyme mix | 3µl |
| ddH20 | 5µl |

(17) Mix gently with finger tapping, centrifuge briefly, incubate at 20°C for 5 minutes, then incubate at 65°C for 5 minutes, add 60 µl of AMPure XP (Beckman) magnetic beads that have been mixed and equilibrated to room temperature, and transfer the mixture to a 1.5 ml centrifuge tube. Invert and mix at room temperature for 5 min;
(18) After instant centrifugation, place the centrifuge tube on a magnetic stand, remove the supernatant once the solution turns clear, and wash twice with 200ul freshly prepared 70% ethanol;
(19) Dry the magnetic beads at room temperature for 2 min, and add 61ul TE buffer, resuspend off the rack and incubate for 2 min, put the centrifuge tube back on the magnetic rack, and take the supernatant after the magnetic beads aggregate;
(20) Prepare a sequencing adapter ligation reaction system in a 1.5ml centrifuge tube as follows:

| Components | Volume |
|---|---|
| DNA | 60µl |
| Ligation buffer (LNB) | 25µl |
| NEBnext Quick T4 DNA Ligase | 10µl |
| Sequencing adapter (AMX) | 5µl |

(21) Close the tube, mix by finger tapping, centrifuge briefly, incubate at room temperature for 10 minutes, and add 40 µl of AMPure XP magnetic beads that have been mixed and equilibrated to room temperature. Continue to invert and mix at room temperature for 5 minutes;
(22) After a brief centrifugation, place the centrifuge tube on a magnetic stand. Once the solution turns clear, discard the supernatant, add 250 µl of short fragment washing buffer (SFB), cover the tube, and mix by finger tapping until the magnetic beads are suspended again. After centrifugation, put the centrifuge tube back on the magnetic rack, and once the solution turns clear, discard the supernatant;
(23) Repeat step 22;
(24) Dry the magnetic beads at room temperature for 30 seconds, add 15 µl of elution buffer (buffer EB), flick off the rack to resuspend, and incubate at room temperature for 10 minutes;
(25) After instant centrifugation, place the centrifuge tube on a magnetic stand, and once the solution turns clear, transfer the supernatant to a 1.5ml tube for future use;
(26) Take 1 µl of DNA for quantification with Qubit reagent. The total amount of target DNA is 1~20 ng/µl. If the DNA concentration is too high, dilute it with elution buffer (Buffer EB).

### 3. Sequencing

(1) Mix a tube of the flowcell priming buffer (FB) with 30 µl of flushing aid (FLT), vortex well, inject 800 µl through the injection hole into the chip, leave it at room temperature for 5 minutes, then open the injection hole (Spot on), and then Inject 200 µl of rinse mix from the initial well;
(2) Prepare the sequencing reaction in a 1.5ml tube according to the table below:

| Components | Volume |
|---|---|
| Sequencing buffer (SQB) | 37.5µl |
| Library Loading beads (LB) | 25.5µl |
| DNA library | 12µl |

(3) After gently pipetting twice with a pipette, load 75 µl of the mixture into the sequencing flowcell through the sample loading hole. After the mixture has completely flowed into the flowcell, cover the sample hole first, and then close the initial hole;
(4) Sample was sequenced until 1Gb of data for each sample were generated.

### 4. Bioinformatics analysis

(1) Raw FAST5 reads files were base-called using the Guppy v3.2.4 basecalling software;
(2) Called fastq files of clinical specimens were analyzed with the EPI2ME WIMP rev.3.3.1 pipeline for pathogen identification, and with Antimicrobial resistance for drug susceptibility prediction;
(3) WIMP analysis showed that *Acinetobacter baumannii* was positive with a total reads of 10096, as listed in Table 20;

**Table 20**

| Species | Reads |
|---|---|
| Corynebacterium striatum | 43890 |
| Homo sapiens | 15657 |
| **Acinetobacter baumannii** | 10,096 |
| Corynebacterium simulans | 9,247 |
| Streptococcus mitis | 3,341 |
| Streptococcus pneumoniae | 1,511 |
| Streptococcus sp. oral taxon 431 | 1,354 |
| Corynebacterium diphtheriae | 1,311 |
| Corynebacterium aurimucosum | 1,056 |
| Corynebacterium resistens | 1,010 |
| Streptococcus pseudopneumoniae | 728 |
| Streptococcus oralis | 621 |

(4) The antimicrobial resistance determinants including sul2, APH(3')-Ia, OXA239, and gyrA(T) identified are listed in Table 21, and their corresponding resistance profiles are inferred as in Table 22;

**Table 21**

| Antimicrobial resistance determinants | |
|---|---|
| abeM | adeL |
| abeS | adeN |
| ADC-22 | adeR |
| adeA | ANT(3")-IIb |
| adeB | APH(3")-Ib |
| adeC | APH(3')-Ia |
| adeF | APH(6)-Id |
| adeG | mphD |
| adeH | msrE |
| adeI | OXA-239 |
| adeJ | sul2 |
| adeK | TEM-122 |
| tet(B) | TEM-90 |
| tetR | |

**Table 22**

| | Antibiotics | | | | | | | | | | | | |
|---|---|---|---|---|---|---|---|---|---|---|---|---|---|
| Gen es | Sulfam ethoxaz ole | Cefta zidi me | Cef epi me | Cef otet an | Cefo xicil lin | Piper acill in | Mero pene m | Imi pen em | Gent ami cin | Tobr amy cin | Ami kac in | Levof loxac in | Cipro floxa cin |
| AM R | Sul2 | OXA239 | | | | | | | APH(3')-Ia | | | gyrA(T) | |
| Phenoty pe | R | R | R | R | R | R | R | R | R | R | R | R | R |

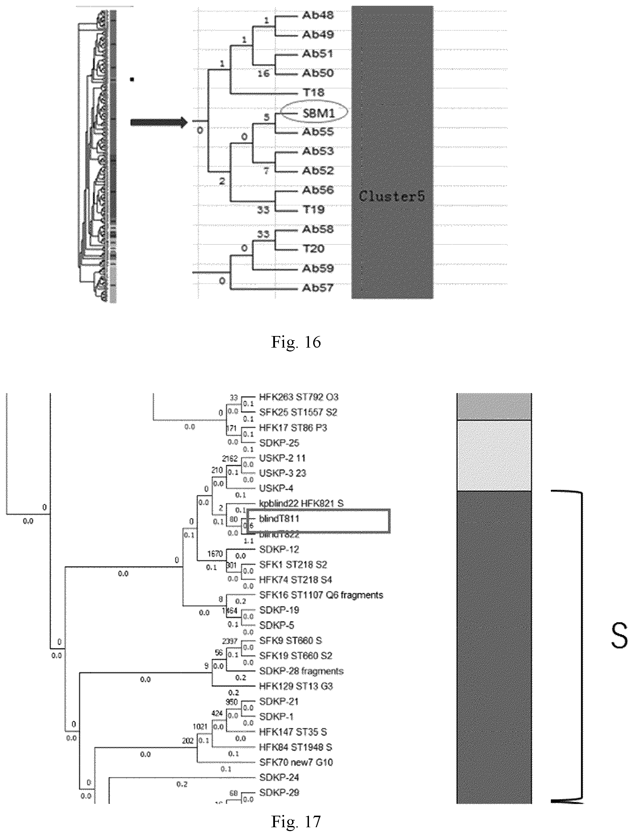

(5) The genome-wide SNPs loci of library *Acinetobacter baumannii* together with this test clinical specimen were identified using kSNP3 (Version 3.1) (Standard mode, kmer=31), and a genomic phylogenetic tree based on SNP similairties were generated for cluster analysis. The test sample was located in the Cluster 5 of phylogenetic tree, as shown in FIG. 16. Therefore, this *Acinetobacter baumannii* strain was determined resistant to all listed antibiotics, as shown in Table 23.

**Table 23**

| | Antibiotics | | | | | | | | | | | | |
|---|---|---|---|---|---|---|---|---|---|---|---|---|---|
| Clu ster | Sulfa metho xazole | Ceft azidi me | Cef epi me | Cef otet an | Cefo xicil lin | Pipe racil lin | Mer open em | Imi pen em | Gent amic in | Tobr amy cin | Am ikac in | Levo floxa cin | Cipro floxa cin |
| 1 | S | S | S | S | S | S | S | S | S | S | S | S | S |
| 2 | R | R | R | R | R | R | R | R | R/I | R/S | R/S | R | R |
| 3 | R | R | R | R | R | R | R | R | R | S /R | S/R | R | R |
| 4 | S | R | R | R | R | R | R | R | R/S | R/S | S | R | R |
| 5 | R/S | R | R | R | R | R | R | R | R/S | R/S | R/S | R | R |
| 6 | R/S | R | R | R | R | R | R | R | R/S | S | S | R | R |
| 7 | S/R | R | R | R | R | R | R | R | R/S | R/S | R/S | R | R |
| 8 | R/S | R | R | R | R | R | R | R | R | R | R | R | R |
| 9 | S/R | R | R | R | R | R | R | R | R | R | R | R | R |
| 10 | R | R | R | R | R | R | R | R | R | R | R | R | R |
| 11 | R | R | R | R | R | R | R | R | R/I | R/S | R /S | R | R |
| 12 | R | R | R | R | R | R | R | R | R | R | R | R | R |
| 13 | R | R | R | R | R | R | R | R | R | R | R | R | R |
| 14 | S | R | R | R | R | R | R | R | R | R | R | R | R |
| 15 | R | R | R | R | R | R | R | R | R | S | S | R | R |
| 16 | R | S | R | R | R | R | R | R | R | S | S | R | R |
| 17 | S | S | S | S | S | S | S | S | S | S | S | S | S |

### Example 12: Rapid identification and antibiotic susceptibility inference with clinical metagenomics based on Nanopore sequencing technology and the PCR library method

A case of a clinical respiratory sputum specimen containing *Klebsiella pneumoniae,* colletect in August 2019, was selected as the representative to illustrate the way a clinical sample is test and analyzed. Other Enterobacteriaceae can refer to this method for pathogen identification and drug susceptibility determination.

### 1. Method

(1) Take 1ml of sputum sample and add the same amount of the Digestion buffer, mix gently at room temperature for 15 mins. Recover 1ml of liquefied sputum into a 1.5ml tube, centrifuge at 12,000 rpm for 5 mins, discard the supernatant, and add 1ml of PBS buffer to fully resuspend the pellet. Transfer the supernatant to a clean 1.5ml tube, centrifuge at 12000×g for 5min, and discard the supernatant;
(2) Add 500 µl 1× PBS to resuspend the pellet, centrifuge at 12000×g for 5 min, and discard the supernatant;
(3) Add 98 µl of ddH2O and 2 µl of 5% saponin to the precipitate, mix by pipetting with a pipette tip, and let it stand for 10 mins at room temperature;
(4) Add 500 µl of 1× PBS, mix by pipetting with a pipette tip, and centrifuge at 12000×g for 5 min. Discard the supernatant;
(5) Add 40µl 1× PBS to resuspend the pellet, prepare the reaction solution according to the following reaction system, mix well and incubate at 37°C for 15min;

| Components | Volume |
|---|---|
| Sample | 40µl |
| 10 x reaction buffer | 5.6µl |
| Thermo DNase | 10µl |
| ddH2O | 0.5µl |

(6) Use the Bacterial Genomic DNA Extraction kit (Tiangen, China) to extract and purify the genomic DNA from the sample, and elute with 50ul TE;
(7) Take 2ul of the extracted genomic DNA for quantification with Qubit reagent.

### 2. Amplification of Antimicrobial resistance determinants

(1) Prepare the reaction solution according to the following table in a 0.2ml PCR tube:

| Components | Volume |
|---|---|
| Taq 2x master mix | 20µl |
| DNA | 5ul |
| Primer mix | 10µl |
| ddH2O | 5µl |
| Nuclease-free water | 40ul |

(2) PCR under the following conditions:

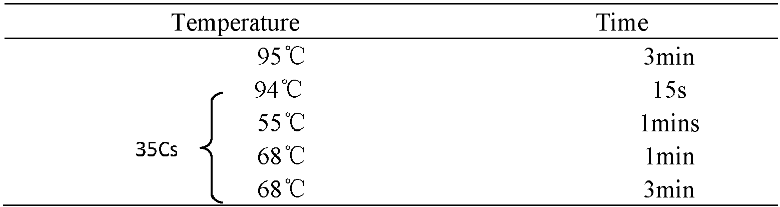

### 3. Library preparation

(1) Prepare the End-prep reaction system according to the following table in a 0.2ml PCR reaction tube:

| Components | Volume |
|---|---|
| DNA | 45µl |
| Ultra II End-prep reaction buffer | 7µl |
| Ultra II End-prep enzyme mix | 3µl |
| ddH20 | 5µl |

(2) Mix gently by finger tapping, centrifuge briefly, incubate at 20°C for 5 mins, and then incubate at 65°C for 5 mins. Add AMPure XP (Beckman) magnetic beads that have been mixed and equilibrated to room temperature, and transfer the mixture to a 1.5ml tube. Invert and mix for 5min;
(3) After instant centrifugation, place the centrifuge tube on a magnetic rack, remove the supernatant after the magnetic beads are enriched, and wash twice with 200ul freshly-prepared 70% ethanol;
(4) Dry the magnetic beads at room temperature for 2 mins, and add 31ul TE buffer. After mixing and incubating for 2 mins, place the tube on the magnetic frame again. After the magnetic beads are aggregated, take the supernatant for use;
(5) Prepare the barcoding reaction solution according to the following table in a 1.5ml tube:

| Components | Volume |
|---|---|
| End-preped DNA | 30µl |
| Barcode Adapter | 20µl |
| Blunt/TA Ligase Master Mix | 50µl |

(6) Gently mix by finger tapping, centrifuge briefly and incubate at room temperature for 10 mins, add 100ul XP magnetic beads, mix by pipetting, and continue to invert and mix at room temperature for 5 minutes;
(7) After instant centrifugation, place the centrifuge tube on a magnetic rack, remove the supernatant after the magnetic beads are enriched, and wash twice with 200ul freshly prepared 70% ethanol;
(8) Dry the magnetic beads at room temperature for 2 min, and add 25ul TE buffer. Resuspend the beads and incubate at room temperature for 2 min, put the centrifuge tube back on the magnetic rack, and take the supernatant after the magnetic beads aggregate;
(9) Take 1ul of the eluate for quantification with Qubit reagent;
(10) Prepare the PCR reaction solution in a 0.2ml PCR tube as follows:

| Components | Volume |
|---|---|
| PCR Barcode 1-96 | 2µl |
| 10 ng/µl adapter ligated template | 2µl |
| Taq 2x master mix | 50µl |
| Nuclease-free water | 46ul |

(11) PCR under the following conditions: 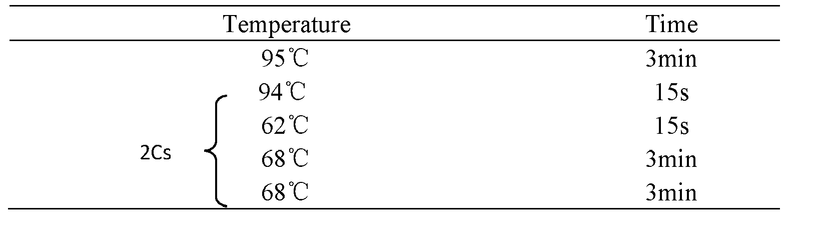
(12) Add 100ul XP magnetic beads, mix by pipetting, and invert and mix at room temperature for 5 minutes;
(13) After instant centrifugation, place the centrifuge tube on a magnetic stand, remove the supernatant once the solution turns clear, and wash twice with 200ul freshly prepared 70% ethanol;
(14) Dry the magnetic beads at room temperature for 2 minutes, add 46ul TE buffer. After mixing and incubating for 2 minutes off the rack, place the centrifuge tube on the magnetic rack again. Once the solution turns clear, take the supernatant for use;
(15) Take 1ul of the eluate for quantification with Qubit reagent;
(16) Prepare an End-prep reaction system in a 0.2ml PCR reaction tube according to the table below:

| Components | Volume |
|---|---|
| DNA | 45µl |
| Ultra II End-prep reaction buffer | 7µl |
| Ultra II End-prep enzyme mix | 3µl |
| ddH20 | 5µl |

(17) Mix gently with finger tapping, centrifuge briefly, incubate at 20°C for 5 minutes, then incubate at 65°C for 5 minutes, add 60 µl of AMPure XP (Beckman) magnetic beads that have been mixed and equilibrated to room temperature, and transfer the mixture to a 1.5 ml centrifuge tube. Invert and mix at room temperature for 5 min;
(18) After instant centrifugation, place the centrifuge tube on a magnetic stand, remove the supernatant once the solution turns clear, and wash twice with 200ul freshly prepared 70% ethanol;
(19) Dry the magnetic beads at room temperature for 2 min, and add 61ul TE buffer, resuspend off the rack and incubate for 2 min, put the centrifuge tube back on the magnetic rack, and take the supernatant after the magnetic beads aggregate;
(20) The product obtained from identification library and the susceptibility library are mixed in equal amounts to form a library-pool;
(21) Prepare a sequencing adapter ligation reaction system in a 1.5ml centrifuge tube as follows:

| Components | Volume |
|---|---|
| DNA mix | 60µl |
| Ligation buffer (LNB) | 25µl |
| NEBnext Quick T4 DNA Ligase | 10µl |
| Sequencing adapter (AMX) | 5µl |

(22) Close the tube, mix by finger tapping, centrifuge briefly, incubate at room temperature for 10 minutes, and add 40 µl of AMPure XP magnetic beads that have been mixed and equilibrated to room temperature. Continue to invert and mix at room temperature for 5 minutes;
(23) After a brief centrifugation, place the centrifuge tube on a magnetic stand. Once the solution turns clear, discard the supernatant, add 250 µl of short fragment washing buffer (SFB), cover the tube, and mix by finger tapping until the magnetic beads are suspended again. After centrifugation, put the centrifuge tube back on the magnetic rack, and once the solution turns clear, discard the supernatant;
(24) Repeat step 23;
(25) Dry the magnetic beads at room temperature for 30 seconds, add 15 µl of elution buffer (buffer EB), flick off the rack to resuspend, and incubate at room temperature for 10 minutes;
(26) After instant centrifugation, place the centrifuge tube on a magnetic stand, and once the solution turns clear, transfer the supernatant to a 1.5ml tube for future use;
(27) Take 1 µl of DNA for quantification with Qubit reagent. The total amount of target DNA is 1~20 ng/µl. If the DNA concentration is too high, dilute it with elution buffer (Buffer EB).

### 4. Sequencing

(1) Mix a tube of the flowcell priming buffer (FB) with 30 µl of flushing aid (FLT), vortex well, inject 800 µl through the injection hole into the chip, leave it at room temperature for 5 minutes, then open the injection hole (Spot on), and then Inject 200 µl of rinse mix from the initial well;
(2) Prepare the sequencing reaction in a 1.5ml tube according to the table below:

| Components | Volume |
|---|---|
| Sequencing buffer (SQB) | 37.5µl |
| Library Loading beads (LB) | 25.5µl |
| DNA library | 12µl |

(3) After gently pipetting twice with a pipette, load 75 µl of the mixture into the sequencing flowcell through the sample loading hole. After the mixture has completely flowed into the flowcell, cover the sample hole first, and then close the initial hole;
(4) Sample was sequenced until 1Gb of data for each sample were generated.

### 5. Bioinformatics analysis

(1) Raw FAST5 reads files were base-called using the Guppy v3.2.4 basecalling software;
(2) Called fastq files of clinical specimens were analyzed with the EPI2ME WIMP rev.3.3.1 pipeline for pathogen identification, and with Antimicrobial resistance for drug susceptibility prediction;
(3) WIMP analysis showed that *Klebsiella pneumoniae* was positive with a total reads of 88638, as listed in Table 24;

**Table 24**

| Species | Reads |
|---|---|
| **Klebsiella pneumoniae** | 88,638 |
| Homo sapiens | 66,519 |
| Escherichia coli | 4,635 |
| Rothiamucilaginosa | 2,917 |
| Streptococcus mitis | 1,044 |
| Acinetobacter baumannii | 1,001 |
| Corynebacterium striatum | 861 |
| Klebsiella variicola | 527 |
| Streptococcus sp. oral taxon 431 | 444 |
| Streptococcus pneumoniae | 374 |
| Veillonellaparvula | 353 |
| Acinetobacter nosocomialis | 310 |

(4) Results of Antimicrobial resistance determinants: none of the antimicrobial resistance determinants including CTX-M-65, TEM-1B, IMP-4, KPC-2, rmtB, AAC(3')-Iid, QRDR, gyrA(T), tetA, tetD, sul1, sul2, sul3 were detected;
(5) The genome-wide SNPs loci of library *Klebsiella pneumoniae* together with this test clinical specimen were identified using kSNP3 (Version 3.1) (Standard mode, kmer=31), and a genomic phylogenetic tree based on SNP similairties were generated for cluster analysis. The test sample was located in the S branch of phylogenetic tree, as shown in FIG. 17. Therefore, this *Klebsiella pneumoniae* strain was determined to be susceptible to all antibiotics involved in this study, which is in agreement with the gold standard VITEK2 AST result.

## Claims

1. A method of determining the drug susceptibility of a pathogen in a test sample,c **characterized by** comprising:
detecting biomarkers in a test sample;
locating the sample in a phylogenetic tree based on biomarker information;
obtaining drug susceptibility prediction rules based on the phylogenetic tree positioning of the sample; and
determining the drug susceptibility of a pathogen according to the prediction rules.

2. The method of of determining the drug susceptibility of a pathogen in a test sample according to claim 1, **characterized in that** the biomarker information is metabolic fingerprints and/or nucleic acid sequences of a pathogen.

3. The method of of determining the drug susceptibility of a pathogen in a test sample according to claim 2, **characterized in that** the metabolic fingerprints are feature information of metabolites detected by mass spectrometry, preferably, the feature information is one or more of mass-to-charge ratio, retention time, and species abundance of the metabolites.

4. The method of of determining the drug susceptibility of a pathogen in a test sample according to claim 3, **characterized in that** the metabolites are water-soluble molecules with a mass-to-charge ratio between 50-1500 Da and a minimum abundance value of 2000.

5. The method of of determining the drug susceptibility of a pathogen in a test sample according to claim 2, **characterized in that** the nucleic acid sequences are antimicrobial resistance determinants in the genome of a pathogen, preferably, the antimicrobial resistance determinants are selected from the group consisting of antibiotic resistance genes, plasmids, chromosomal housekeeping genes, insertion sequences, transposons and integrons.

6. The method of of determining the drug susceptibility of a pathogen in a test sample according to claim 5, **characterized in that** the antibiotic resistance genes are selected from the group consisting of *abarmA, abAPH(3')-Ia, abOXA239, abNDM-10, abgyrA, abSUL-1, abSUL-2, abSUL-3, kpCTX-M-65, kpTEM-1b, kpIMP-4, kpKPC-2, kprmtB, kpAAC(3')-Iid, kpQNR-S, kpgyrA, kpparC, kptetA, kptetD, kpSUL-1, kpSUL-2, kpSUL-3, ecrmtB, ecAAC(3')-Iid, ecgyrA, ectetA, ectetB, ecSUL-1, ecSUL-2, ecSUL-3, ecIMP-4, ecNDM-5, ecTEM-1b, ecCTX-M-14, ecCTX-M-55, ecCTX-M-65, ecCMY, paTEM-1b, paGES-1, paPER-1, paKPC-2, paOXA-246, parmtB, paAAC(3')-Iid, paAAC(6')-IIa, paVIM-2, pagyrA, efermB, eftetM, eftetL, efparC, efANT(6')-Ia, stmecA, stmsrA, stermA, stermB, stermC, strpoB, stgyrA, stAAC(6')-APH(2"*), *stdfrG, sttetK, sttetL, stcfrA, spbpb1a, sppbp2x, spbpb2b, spdfr, sptetM, spermB, spgyrA, aat1a, acc1, adp1, mpib, sya1, vps13, zwflb,fcy2, furl, fca1, erg11, erg3, tacl, cdr1, cdr2, mdr1, pdr1, upc2a, fks1hs1, fks1hs2, fks2hs1, fks2hs2.*

7. The method of of determining the drug susceptibility of a pathogen in a test sample according to claim 1, **characterized in that** the phylogenetic tree is obtained by liquid chromatography-tandem mass spectrometry technology and/or whole genome sequencing technology.

8. The method of of determining the drug susceptibility of a pathogen in a test sample according to claim 1, **characterized in that** the phylogenetic tree is selected from the group consisting of a metabolic spectrum phylogenetic tree constructed based on the species and amounts of metabolites, a whole genome phylogenetic tree constructed based on SNPs and InDels, and a core genome phylogenetic tree constructed based on antimicrobial resistance determinants and their upstream regulatory sequences.

9. The method of of determining the drug susceptibility of a pathogen in a test sample according to claim 8, **characterized in that** the drug susceptibility prediction rules comprise: different metabolite-based prediction rules are applied for different branches of the metabolic spectrum phylogenetic tree, and/or different sequence-based prediction rules are applied for different branches of the genomic phylogenetic tree.

10. The method of of determining the drug susceptibility of a pathogen in a test sample according to claim 9, **characterized in that** the metabolite-based prediction rules are selected from:
1) use independent prediction rules in the drug susceptibility determination of non-fermentative Gram-negative bacteria: when the test sample contains antimicrobial resistance determinants, follow the principle that the drug resistance profile predicted by antimicrobial resistance determinants is preferred over phylogenetic tree interpretation; when the test sample is located in the susceptibility branch of the metabolic spectrum phylogenetic tree, follow the principle that the drug resistance profile inferred by the phylogenetic tree is preferred over antimicrobial resistance determinants analysis and the sample is directly determined to be susceptible to all β-lactam antibiotics;
2) use independent prediction rules in the drug susceptibility determination of Enterobacteriaceae: when the test sample contains antimicrobial resistance determinants, follow the principle that the drug resistance profile predicted by antimicrobial resistance determinants is preferred over phylogenetic tree interpretation; when the test sample is located in the susceptibility branch of the metabolic spectrum phylogenetic tree, follow the principle that the drug resistance profile inferred by the phylogenetic tree is preferred over antimicrobial resistance determinants analysis and the sample is directly determined to be susceptible to β-lactams, β-lactamase inhibitors and cephamycins;
3) use independent prediction rules in the drug susceptibility determination of Gram-positive cocci: when the test sample contains antimicrobial resistance determinants, follow the principle that the drug resistance profile predicted by antimicrobial resistance determinants is preferred over phylogenetic tree interpretation; when the test sample is located in the susceptibility branch of the metabolic spectrum phylogenetic tree, follow the principle that the drug resistance profile inferred by the phylogenetic tree is preferred over antimicrobial resistance determinants analysis and the sample is directly determined to be susceptible to penicillins, macrolides, lincosamides, quinolones, aminoglycosides, glycopeptides and oxazolidinones; when the test sample is identified as *Enterococcus faecalis* and has the metabolic fingerprints of a sequence type 4 *Enterococcus faecalis* clone, the sample is directly determined to be resistant to penicillins;
4) use independent prediction rules in the drug susceptibility determination of *Streptococcus pneumoniae:* when the test sample contains antimicrobial resistance determinants, follow the principle that the drug resistance profile predicted by antimicrobial resistance determinants is preferred over phylogenetic tree interpretation; when the test sample is identified as *Streptococcus pneumoniae* and has the metabolic fingerprints of a *Streptococcus pneumoniae* clone with altered penicillin-binding protein patterns, the sample is directly determined to be resistant to penicillins; and/or
5) use independent prediction rules in the drug susceptibility determination of Fungi: strictly follow the principle that the drug resistance profile of a fungal strain is inferred on the basis of its closest relatives in the metabolic spectrum phylogenetic tree.

11. The method of of determining the drug susceptibility of a pathogen in a test sample according to claim 9, **characterized in that** the sequence-based prediction rules are selected from:
1) resistance of Enterobacteriaceae to carbapenems and quinolones is determined by the positioning of a test sample in the genomic phylogenetic tree, following the principle that the drug resistance profile predicted by phylogenetic tree interpretation is preferred over antimicrobial resistance determinants analysis; resistance of Enterobacteriaceae to others is determined by antimicrobial resistance determinants analysis;
2) resistance of non-fermentative Gram-negative bacteria to cephalosporins and carbapenems is determined by the positioning of a test sample in the genomic phylogenetic tree, following the principle that the drug resistance profile predicted by phylogenetic tree interpretation is preferred over antimicrobial resistance determinants analysis; resistance of non-fermentative Gram-negative bacteria to others is determined by antimicrobial resistance determinants analysis;
3) resistance of Gram-positive cocci to penicillin, ampicillin, oxacillin and cefoxitin is determined by the positioning of a test sample in the genomic phylogenetic tree, following the principle that the drug resistance profile predicted by phylogenetic tree interpretation is preferred over antimicrobial resistance determinants analysis; resistance of Gram-positive cocci to others is determined by antimicrobial resistance determinants analysis;
4) resistance of *Streptococcus pneumoniae* to penicillins and cephalosporins is determined by the positioning of a test sample in the genomic phylogenetic tree, following the principle that the drug resistance profile predicted by phylogenetic tree interpretation is preferred over antimicrobial resistance determinants analysis; resistance of *Streptococcus pneumoniae* to others is determined by antimicrobial resistance determinants analysis; and/or
5) resistance of Fungi to triazoles and amphotericin B formulations is determined by the positioning of a test sample in the genomic phylogenetic tree, following the principle that the drug resistance profile predicted by phylogenetic tree interpretation is preferred over antimicrobial resistance determinants analysis; resistance of Fungi to others is determined by antimicrobial resistance determinants analysis.

12. Application of the phylogenetic tree of a pathogen in the preparation of an antimicrobial susceptibility diagnostic product, **characterized in that** the phylogenetic tree is obtained by liquid chromatography-tandem mass spectrometry technology and/or whole genome sequencing technology.

13. The application of determining the drug susceptibility of a pathogen in a test sample according to claim 12, **characterized in that** the phylogenetic tree is selected from the group consisting of a metabolic spectrum phylogenetic tree constructed based on the species and amounts of metabolites, a whole genome phylogenetic tree constructed based on SNPs and InDels, and a core genome phylogenetic tree constructed based on antimicrobial resistance determinants and their upstream regulatory sequences.

14. The application of determining the drug susceptibility of a pathogen in a test sample according to claim 12, **characterized in that** the antimicrobial susceptibility diagnostic product further comprises reagent and equipment for detecting the biomarker information in a test sample.

15. The application of determining the drug susceptibility of a pathogen in a test sample according to claim 14, **characterized in that** the equipment for detecting the biomarker information is selected from the group consisting of liquid chromatography-tandem mass spectrometry and whole genome sequencing devices.

16. The application of determining the drug susceptibility of a pathogen in a test sample according to claim 14, **characterized in that** the reagent for detecting the biomarker information is selected from the group consisting of a pathogen identification and drug susceptibility diagnostic kit based on liquid chromatography-tandem mass spectrometry, and a pathogen identification and drug susceptibility diagnostic kit based on whole genome sequencing technology.

17. A pathogen identification and drug susceptibility diagnostic kit, characterize by comprising:
KIT1: a pathogen identification and drug susceptibility diagnostic kit based on liquid chromatography-tandem mass spectrometry; or,
KIT2: a pathogen identification and drug susceptibility diagnostic kit based on whole genome sequencing technology; and,
the phylogenetic tree of a pathogen in the test sample.

18. The pathogen identification and drug susceptibility diagnostic kit according to claim 17, **characterized in that** the pathogen identification and drug susceptibility diagnostic kit based on liquid chromatography-tandem mass spectrometry comprise bacterial standards, fungal standards, extraction buffer and resuspension buffer.

19. The pathogen identification and drug susceptibility diagnostic kit according to claim 17, **characterized in that** pathogen identification and drug susceptibility diagnostic kit based on whole genome sequencing technology comprise cell lysis reagents, primer mixture, target enrichment reagents, library preparation reagents, native barcoding reagents and sequencing reagents.
